# EUROPEAN PATENT SPECIFICATION

(11) **EP 4 654 896 B1**
(45) Date of publication and mention of the grant of the patent: **28.10.2026**
(21) Application number: 23837586.9
(22) Date of filing: 20.12.2023
(51) Int. Cl.: A61B 8/12, A61B 5/00, A61B 8/00, A61B 8/08

(54) **TORSIONAL ULTRASONIC WAVES TRANSDUCER AND MEDICAL DEVICE THEREOF**
MEDIZINISCHE VORRICHTUNG MIT TORSIONSULTRASCHALLWANDLER
DISPOSITIF MÉDICAL DE TRANSDUCTEUR D'ONDES ULTRASONORES DE TORSION

(30) Priority: 23.01.2023 ES 202330102 U; 31.01.2023 ES 202330141 U
(43) Date of publication of application: 03.12.2025
(73) Proprietor: ULTRASOUND INNOVATION MEDTECH S.L., 48160 Derio - Bizkaia (ES)
(72) Inventor: GARCÍA SALGUERO, Alberto, 48160 Derio - Bizkaia (ES); CORBETTA, Francesco, 48160 Derio - Bizkaia (ES); BORJAS MORENO, Jordi, 48160 Derio - Bizkaia (ES)
(74) Representative: Hoffmann Eitle
(86) International application number: PCT/EP2023/086898
(87) International publication number: WO 2024/156446

(56) References cited:
- EP-A1- 3 034 006
- CN-A- 108 324 322
- CN-A- 113 520 466
- CN-A- 115 493 752
- JP-A- 2009 201 989
- US-A1- 2017 303 894
- US-A1- 2018 214 913

## Description

### Technical field of the invention

The present invention belongs to the field of medical devices. Particularly, the present invention relates to a medical device comprising a torsional ultrasonic waves transducer configured to contact a specimen enabling the best reading from the torsional ultrasonic waves transducer. It also relates to a device thereof configured for the arrangement of a membrane in the medical device.

### Background of the invention

Torsional waves are a spatial distribution of transverse waves propagating along an axis in which particle movement occurs along a circumference the centre of which is said axis, such that the amplitude of the movement in the generating plane is proportional to the distance from the axis within the diameter of the transducer.

These waves propagate through solid and semi-solid media, but not through perfect liquids, so measuring the speed of sound in media of this type can be very useful for studying their structural characteristics of input energy into another different type of output energy. These devices include, among others, electromechanical transducers which transform electrical energy into mechanical energy in the form of bidirectional displacements elastically coupled with stresses.

Ultrasonic transducers emit and receive ultrasonic waves, which allows, based on solid mechanics, identifying changes in tissue consistency which may indicate the presence of tumours, and quantifying mechanical or physical changes in the tissue can anticipate certain pathologies sooner than other diagnostic techniques can.

Because torsional waves (TW) are a contact technology and quantify mechanical parameters of the surface being measured, the values obtained can vary quickly when the properties of the surface are altered. This can lead to erroneous measurements and inconsistencies between measurements, which prevents from torsional waves technologies to be used in a reliable way in a medical environment.

There is therefore a need for torsional ultrasonic waves transducer technology capable of ensuring that the properties of the surface being contacted are not altered or altered as little as possible. According to its summary section, the document JP 2009 201989 A discloses systems and methods for determining tissue properties such as its shear, viscoelastic and/or modulus information.

### Summary of the invention

A medical device configured to contact a specimen through its distal end, the medical device comprising : a) a torsional ultrasonic waves transducer located at the distal end of the medical device and configured to induce a torsional wave that goes through said specimen, b) a force sensor configured to determine the force with which the medical device contacts the specimen, and c) a processing unit. The torsional ultrasonic waves transducer is configured to contact the specimen with a predetermined range of force and the processing unit is configured to determine whether the force with which the medical device contacts the specimen is within the predetermined range of force. The specimen is the cervix of a uterus and the torsional ultrasonic waves transducer is configured to contact the cervix with a range between 0 and 200 g/cm². It also relates to a device thereof configured for the arrangement of a membrane in the medical device.

In a preferred embodiment, the medical device further comprises feedback means. The feedback means are configured to provide feedback to the user of the medical device about the force with which the device contacts the specimen. More preferably, the feedback means are configured to further provide feedback to the user of the medical device about whether the force is within the predetermined range of force or not. In another more preferred embodiment, the feedback means are located at the proximal end of the medical device.

In another more preferred embodiment, the medical device further comprises an imaging mean and the feedback means comprise a visual feedback device configured to display the images captured by the imaging means. More preferably, the imaging means are located at the distal end of the medical device, and wherein the imaging means and the visual feedback device are coaxially located within the medical device. Even further preferably, the imaging means and the visual feedback device are coaxially within the medical device along the axis formed by the distal end and proximal end.

In another preferred embodiment, the force sensor is comprised within the distal section of the medical device.

In another preferred embodiment, the force sensor is located perpendicularly to the surface of the medical device configured to contact the specimen.

In another preferred embodiment, the force sensor is comprised between a proximal section of the medical device and a distal section of the medical device. In a particular embodiment, the force sensor determines the force with which the device contacts the specimen based on the relative position of the distal section (180) with respect to the proximal section. More preferably, the force sensor determines the force with which the device contacts the specimen when the distal section is displaced longitudinally relative to the proximal section.

In another particular embedment, the force sensor is preloaded and determines the force with which the device contacts the specimen according to the release of pressure on the specimen when the distal section is displaced longitudinally with respect to the proximal section.

In another preferred embodiment, the force sensor further comprises a protection system configured to prevent the force sensor from getting damaged when a force exceeding the working range is applied to the distal end of the medical device, preferably wherein the protection system comprises a spring.

In another preferred embodiment, the medical device further comprises a handle on its proximal end configured to be held by a hand and with an angle comprised between 85° and 120° with respect to the axis formed by the distal end and proximal end.

In another preferred embodiment, the processing unit is configured to discard the readings of torsional ultrasonic waves transducer if the force applied is not within the predetermined range of force.

In another preferred embodiment, the processing unit is configured to prevent the torsional ultrasonic waves transducer from inducing torsional waves if the force applied is not within the predetermined range of force.

In another preferred embodiment, the torsional ultrasonic waves transducer comprises an emitter device for emitting torsional ultrasonic waves. The emitter device comprises an electrical signal generator connected to an electromechanical actuator which is in turn attached to a contact element that comes into contact with a specimen, such that when the actuator receives electrical signals, it induces rotational movement of the contact element, and when said contact element comes into contact with the specimen, it induces a torsional wave that goes through said specimen, wherein the torsional ultrasonic waves transducer further comprises means for receiving the distorted signal after it goes through the specimen.

In a more preferred embodiment, the means for receiving the distorted signal comprise two or more piezoelectric elements located equidistant from one another and placed between two rings made from a non-conducting material and in that the axis of rotation of the rings coincides with the axis of rotation of the electromechanical actuator. In another more preferred embodiment, an attenuating element, preferably an attenuating material with a shore A lower than 80, is fixed to the outer face of the ring that is the farthest away from the area of contact with the specimen. In another more preferred embodiment, the contact element has a considerably frustoconical shape such that its smaller base is attached to the electromechanical actuator and its larger base is arranged at the distal end of the transducer of the invention so that it comes into contact with the specimen on which shear wave is to be transmitted.

In another more preferred embodiment, the electromechanical actuator is covered by a Faraday cage which eliminates electronic noise. In another more preferred embodiment, the outer face of one of the rings and the surface of the element of the emitter device that comes into contact with the specimen are located on the same plane.

In another more preferred embodiment the polarization of the piezoelectric elements is perpendicular to the axis of rotation of the rings in the radial direction.

In another preferred embodiment, the medical device further comprises: d) a conduit to connect the outside to the inner space of the medical device, the conduit connected to the outside through an opening; e) a sealing element at the opposite end of the conduit, configured to control the passage of air between the inner space and the conduit; and f) a vacuum pump connected to the sealing element and configured to draw air from the opening through the conduit. The medical device is configured to receive a membrane on its outside covering the opening, so that the vacuum pump is configured to extract the air between the sealing element and the membrane.

In a more preferred embodiment, the vacuum pump is connected to a pressure sensor configured to measure the pressure in the conduit, and where the vacuum pump is configured to maintain a minimum pressure in the space between the membrane and the sealing element as measured by the pressure sensor. More preferably, the minimum pressure is comprised between 700 and 800 mBar.

In another more preferred embodiment, the medical device is essentially elongated, and configured to contact the specimen through the distal section, and where the device is configured so that the membrane covers at least the distal section. In another more preferred embodiment, the opening is encompassed in the distal end of the medical device. In another more preferred embodiment, the medical device is configured to receive a prophylactic as a membrane.

In another more preferred embodiment, the medical device comprises at least one indentation in its outer surface configured to receive the edges of the membrane. More preferably, the indentation is configured to receive the edges of the membrane in a sealed form.

In another preferred embodiment, the medical device is a probe. More preferably, the medical device is a uterine tube and the specimen is the cervix of a uterus.

Another aspect of the invention refers to a device configured for the arrangement of a membrane in a probe, the device being essentially elongated, configured to receive a probe longitudinally and comprising a proximal zone and a distal zone, comprising in its proximal zone: a) opening configured for the insertion of probe and; b) at least one indentation defined by its radial outer surface and configured to receive the edge of a membrane covering the opening. The membrane is essentially longitudinally elongated, such that the edge of the membrane comprises part of the membrane.

In a preferred embodiment, the probe comprises a distal end through which it is introduced into device and wherein the device further comprises a distal end in its distal area which in turn comprises an inner surface configured to receive the distal end of the probe. More preferably the inner surface of the distal end of device comprises the complementary shape of the distal end of probe, configured to arrange the membrane in contact with the distal end of probe evenly.

In another preferred embodiment, the distal end of the device is configured to be decoupled from the rest of the device and replaced by another distal end of the device according to the distal end of the probe.

In another preferred embodiment, the opening is configured to fit the respective surface of the probe when the probe is fully inserted into the device.

In another preferred embodiment, the indentation is configured to allow the release of the membrane once the probe has been inserted to the distal end of the device.

In another preferred embodiment, the device is configured to arrange the membrane in a probe which in turn comprises in its proximal part an indentation configured to receive the edge of the membrane once the membrane has already been placed on the probe.

In another preferred embodiment, the opening is essentially circular, and the indentation defines a circumference with a diameter between 5 and 50 mm.

In another preferred embodiment, the device comprises at least one lateral opening configured to allow visualization of the membrane arrangement over the probe.

In another preferred embodiment, the device is configured to arrange the membrane in a uterine probe. More preferably, the device is configured to arrange membrane in a torsional wave uterine probe, preferably in a torsional ultrasonic wave probe.

In another preferred embodiment, the device where device comprises a handle at its distal end.

In yet another preferred embodiment, the membrane (70) is a prophylactic.

In another preferred embodiment, the device also comprises in its proximal area at least one magnet configured to attract at least one ferromagnetic element or magnet comprising in the proximal part of the probe.

### Brief description of the drawings

To enable a better understanding of the present disclosure, and to show how the present disclosure may be carried out, reference will now be made, by way of example only, to the accompanying schematic drawings, wherein:
Fig. 1A shows a perspective view of the distal section of a medical device according to one or more embodiments of the invention.
Fig. 1B shows a lateral view of the internal components disposition within the distal section of a medical device according to one or more embodiments of the invention.
Fig. 2 shows a perspective view of a medical device according to one or more embodiments of the invention.
Fig. 3 shows a lateral view of the medical device of Fig. 2 according to one or more embodiments of the invention.
Fig. 4 shows a close up view of the distal end of a medical device according to one or more embodiments of the invention.
Fig. 5 shows the proximal end of a medical device according to one or more embodiments of the invention.
Fig. 6 shows a visual feedback device interface according to one or more embodiments of the invention; before use (A), in use but before torsional waves are induced through the specimen (B), in use while torsional waves are induced through the specimen (C), and after torsional waves have been induced through the specimen (D).
Fig. 7 shows a diagram of the force sensor mechanism of a device according to one or more embodiments of the invention.
Fig. 8 shows the emitter device of the torsional ultrasonic waves transducer according to one or more embodiments of the invention.
Fig. 9 shows the contact element of the torsional ultrasonic waves transducer according to one or more embodiments of the invention.
Fig. 10 shows a section of the receiver device of the torsional ultrasonic waves transducer according to one or more embodiments of the invention in which the rings and the piezoelectric elements can be seen.
Fig. 11 shows the arrangement of the emitter and the receiver devices of the torsional ultrasonic waves transducer according to one or more embodiments of the invention in which the contact element, the rings and the piezoelectric elements can be seen.
Fig. 12 schematically depicts the contact between the transducer and the specimen according to one or more embodiments of the invention.
Fig. 13 depicts a piezoelectric element (5) and the direction of polarization (P) thereof according to one or more embodiments of the invention.
Fig. 14 shows a section of a transducer according to one or more embodiments of the invention in which the arrangement of the emitter device with respect to the receiver device can be seen, and the arrangement thereof inside a casing together with the attenuating elements.
Fig. 15 shows a perspective view of a medical device according to one or more embodiments of the invention.
Fig. 16 shows another perspective view of a medical device with the distal and proximal sections in exploded view according to one or more embodiments of the invention.
Fig. 17A shows a longitudinal section of a device according to one or more embodiments of the invention.
Fig. 17B shows a simplified view of the longitudinal section of the distal section of a device according to one or more embodiments of the invention.
Fig. 18A shows a perspective view of a device according to one or more embodiments of the invention.
Fig. 18B shows a perspective view of the device in Fig. 18A with a membrane according to one or more embodiments of the invention.
Fig. 19A shows another perspective view of the device in Fig. 18A with a membrane and probe for which it is configured to receive according to one or more embodiments of the invention.
Fig. 19B shows a longitudinal section of the device in Fig. 18A with a membrane and a probe for which it is configured to receive according to one or more embodiments of the invention.
Fig. 20A shows a perspective view of the device in Fig. 18A, with the probe fully inserted and the membrane fully arranged on it, according to one or more embodiments of the invention.
Fig. 20B shows a longitudinal section of the device in Fig. 18A, with the probe fully inserted and the membrane fully arranged on it, according to one or more embodiments of the invention.
Fig. 21A shows a perspective view of the device in Fig. 18A and a probe once it has been removed from the device, according to one or more embodiments of the invention.
Fig. 21B shows a longitudinal section of a probe with a membrane arranged on its surface once it has been extracted from a device, according to one or more embodiments of the invention.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together.

Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "specimen" in the context of the present invention refers preferably to a biological specimen and particularly to any type of biological tissue, whether in-vivo or in-vitro. For example, the specimen may be a sample taken from an animal, or a part of the animal such as a section of skin, a limb, the inside of a cavity, a mucosa, or any other part of an animal or human. Thus, the specimen must be understood as the material, preferably a tissue, a tissue culture or a cell culture, through which waves emitted by the transducer are made to pass in order to learn about its structural characteristics (elastic parameters, viscoelastic parameters, microstructural geometry, porous, or energy dissipation models, among others).

The term "medical device" in the context of the present invention preferably refers to any type of instrument, device, or equipment for the diagnosis, prevention, monitoring, prediction, prognosis, treatment, relief, or research of an animal, particularly human beings.

The term "probe" in the context of the present invention refers preferably to an essentially tubular device, which allows the examination, diagnosis and/or provision of a therapy through a conduit or cavity of the body.

The term "membrane" in the context of the present invention refers preferably to a thin, generally flexible and preferably elastic film that may be organic or inorganic and comprise materials such as nitrile, latex or polyurethane.

The term "opening" in the context of the present invention refers preferably to a hole in the surface of the device that allows access to the interior of the device.

The term " indentation" in the context of the present invention refers preferably to a narrow, elongated area on a surface that is depressed in front of the rest of the surface, generating a concave zone in it.

The term "edge" in the context of the present invention, and particularly referring to the membrane, refers preferably to the ends of the membrane.

The term "essentially circular" in the context of the present invention refers preferably to a shape which in its general appearance resembles in shape a circle, although it may have irregularities at the edges of the circle, or is partially truncated, or slightly distorted on one or more axes.

The term "electromechanical actuator" is preferably understood as a device capable of transforming electrical energy into a movement, particularly a rotational movement. In a particular embodiment suitable for this invention, the electromechanical actuator is stimulated with an electrical signal generated by an electrical pulse generator and is capable of transforming said signal into a minimum fraction of a rotation which will be used to generate the wave that is subsequently analysed. An example of actuators of this type may consist of an electromagnetic motor. For the purpose of the present invention, the electromechanical actuator is preferably stimulated by means capable of generating electrical signals or waves, hereinafter "electrical signal generator."

The term "electrical signal" is preferably understood as an electrical magnitude the value of which depends on time. For the purpose of the present invention, constant magnitudes will be considered as particular cases of electrical signals. The electrical signals generated by an electrical signal generator can be periodic signals (sine, square, triangular, "sawtooth"-shaped, etc.). Therefore, by connecting the generator to an actuator which transforms the signal into a rotational movement, said actuator rotates a minimum fraction of a turn depending on the voltage, frequency and/or time between pulses determined by the signal. Any electronic circuit digitalizing the electrical signals at the desired frequencies can be used as the electrical signal generator. Another example of an electrical signal generator used in the experimental designs of the present invention may be an oscilloscope, as it allows emitting an electrical signal with a variable voltage over a specific time.

The term "Biocompatible material" is preferably understood as a material the composition of which does not interfere with or causes degradation of the biological medium in which it is used. These materials are typically used for making devices or elements thereof which must be in direct temporary or prolonged contact with the internal fluids and tissues of the body, such as probes, syringes, prostheses, etc. An example of this material is polylactic acid (PLA).

The term "contact element" refers to the part or element that is located in the distal or front part of the transducer and comes into contact with the specimen on which the wave is to be transmitted. The surface of the contact element coming into contact with the specimen must be considerably flat to allow suitable wave transmission.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

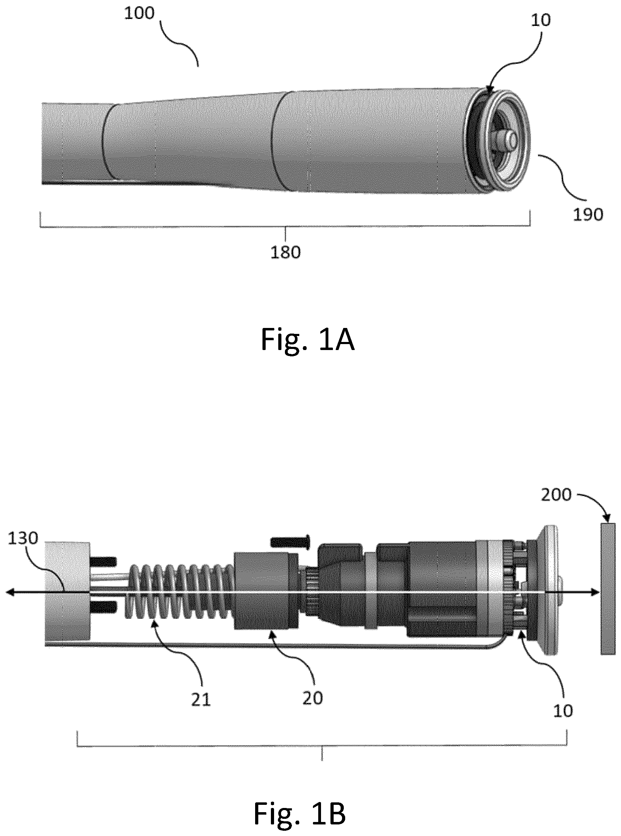

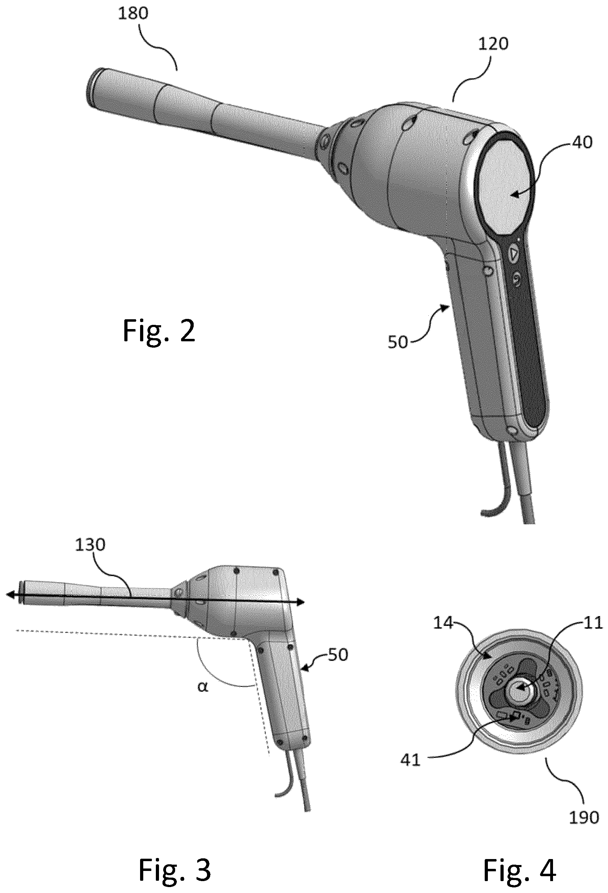

A first aspect of the invention, as shown with respect to Figs. 1A, 1B and 2, relates to a device (100) configured to contact a sample (200) through its distal end (190). The device (100) comprises a torsional ultrasonic waves transducer (10), a force sensor (20) and a processing unit (30).

It is noted the present invention is not particularly limited to certain types of samples, and these may comprise either organic or inorganic samples, such as biological samples or specimens. From herein after, reference will now be made, by way of example only, to a specimen (200). Moreover, it is according to a preferred embodiment of any of the embodiments of the first aspect of the invention that the sample (200) is a specimen (200).

The torsional ultrasonic waves transducer (10) is located at the distal end (190) of the device (100) and configured to induce a torsional wave that goes through said specimen (200). The distal end (190) is preferably understood as the further part of the device (100) from wherein the user controls the device. The torsional ultrasonic waves transducer can induce torsional waves through various mechanisms, providing flexibility in applications. Possible induction methods include mechanical means, where the transducer physically imparts a rotational force to the medium, or electromagnetic means, where magnetic fields generate the required torsional motion. These methods will be elaborated further below.

The force sensor (20) is configured to determine the force with which the device (100) contacts the specimen (200).

The force sensor (20) can be of many types and can be similar or the same as the pressure sensor (67) or not, for example a piezoresistive, capacitive, electromagnetic, piezoelectric, strain gauge, optical or potentiometric extensometer, among others. Therefore, the force sensor (20) of the present invention is not limited to any particular type of force sensor. Depending on the technology, the force sensor can determine applied forces through diverse methods. This includes measuring the deformation of materials, capturing changes in electrical properties, or quantifying pressure variations. The force sensor may be located in any place within the device (100). It may be strategically located at various points, depending on the device's architecture.

The processing unit (30) may in turn comprise one or more processing units, such as a microprocessor, GPU, CPU, multi-core processor or the like. There is therefore no particular limitation in the processing unit (30) implementation. In certain configurations, the processing unit may be strategically located within the device itself. For instance, it could be positioned alongside the force sensor, contributing to real-time data processing and immediate response to mechanical interactions. This on-board processing enhances the device's autonomy and responsiveness. Alternatively, the processing unit may be part of a broader computing infrastructure. It could be integrated into an external computing device, such as a personal computer (PC) or a remote PC. In this scenario, the device benefits from the processing capabilities of the external computing unit, enabling more complex computations and data analysis, such as aggregate data from other devices (100).

The torsional ultrasonic waves transducer (10) is configured to contact the specimen (200) with a predetermined force or range of force. In a preferred embodiment, torsional ultrasonic waves transducer (10) is configured to contact the specimen (200) with a predetermined range of force.

The term "configured to contact with a predetermined force" in the context of the present invention is preferably understood as the torsional ultrasonic waves transducer (10) being configured to be used within a specific application, this is, to contact the specimen (200) the device (100) is configured to contact. Thus, the torsional ultrasonic waves transducer's configuration is adaptable, specifically tailored to the application and the nature of the specimen (200). Depending on the specimen (200) the torsional ultrasonic waves transducer (10) may be disposed and configured to optimally induce torsional waves through the specimen at the predetermined range of force. Thus, depending on the specimen the device (100) is configured to contact, this range of force may be different.

The preferred force applied by the torsional ultrasonic waves transducer can be set as either a desired singular value or desired defined range. This feature accommodates diverse measurement scenarios, offering versatility in force application.

To provide a concrete example, when the device (100) is a medical device used for cervical examinations as a uterine probe, the force applied by the torsional ultrasonic waves transducer can be set within specified ranges, for instance a range between 0 to 200 g/cm².

The processing unit (30) is configured to determine whether the force with which the device contacts the specimen (200) is within the predetermined range of force.

This determination is achieved through a systematic comparison between the force measured by the force sensor (20) and the range of force the torsional ultrasonic waves transducer (10) is configured to contact the specimen (200).

Advantageously, the device (100) is able to monitor the force with which the device contacts the specimen (200), which means that the user can have a better understanding on the use of the device (100) and determine if he/she shall change the way the device (100) is contacting the specimen (200).

As can be seen, Figs. 1A, 1B and 2 show a device (100) that includes many other additional features, which may not be included in other embodiments or which may be understood in different ways. For example, in Figs. 1A and 1B it can be seen that device (100) has an essentially elongated shape, but other devices (100) according to this invention may not be essentially elongated. In both figures it can be seen that the device (100) comprises a particular torsional ultrasonic waves transducer (10), with a particular distal end shape. However, in other embodiments the device (100) may comprise any other type of torsional ultrasonic waves transducer (10).

It can also be seen in Fig. 2 that device (100) comprises a particular shape, but other devices (100) according to this invention may encompass a different shape. Also it can be seen that the device (100) of Fig. 2 comprises a feedback means (40) a handle (50) and a power input (60). However, in other embodiments, according to the first aspect of the invention, the device (100) may not comprise any of these.

It can also be seen that the distal section (180) is narrower than the proximal section (120), and that the proximal section (120) comprises almost all the electronics of the device (100). However, in other embodiments, the device may comprise other shapes, and the proximal (110) and distal (190) sections have a similar, same, or different shape depending on the application of the device itself (100). In this sense, the present invention is not limited to any particular form of the device (100).

It is also noted that the device (100) may comprise multiple electronic means in order for the processor (30) be able to carry out all its functions according to one or mor embodiment of the present invention. It may further comprise an energy source such as an input power cable or a battery unit configured to feed the electronics. It may further comprise a data transfer unit, either physical or wireless such as Bluetooth or Wi-Fi.

According to a preferred embodiment of any of the embodiments of the first aspect of the invention, the device (100) is a medical device (100). From herein after, reference will now be made, to a medical device (100), although any of the embodiments of the first aspect of the invention are not limited to a medical device (100).

According to a particularly preferred embodiment, the device (100) is a medical device, the specimen (200) is the cervix of a uterus and the torsional ultrasonic waves transducer (10) is configured to contact the cervix (200) with a range between 0 and 200 g/cm².

According to preferred embodiment, the torsional ultrasonic waves transducer (10) is configured to contact the cervix (200) with a range between 50and 200 g/cm², more preferably between 100 and 150 g/cm² or about 100 g/cm².

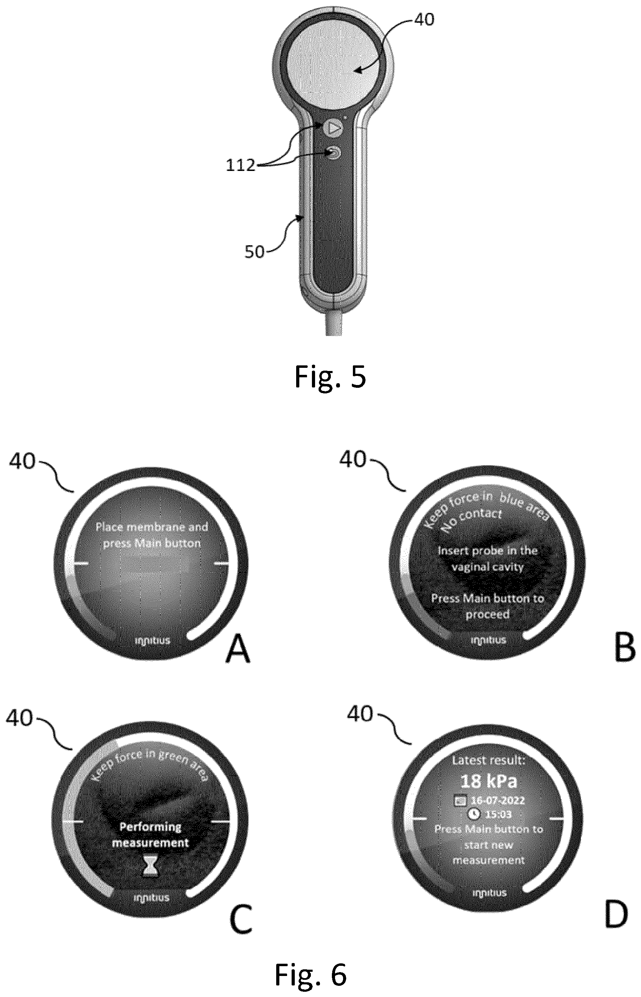

In a particular embodiment of the first aspect of the invention, as shown in Figs 6A to 6D, the medical device (100) further comprises feedback means (40), wherein the feedback means (40) are configured to provide feedback to the user of the medical device (100) about the force with which the device contacts the specimen (200).

It is noted the feedback means (40) may encompass a range of different devices designed to convey force-related information to the user, such as visual means (e.g. screens, LEDs, light indicators, etc), haptic means or auditory means among others.

It is noted that using the feedback means, the force with which the medical device (100) contacts the specimen (200) can be provided to the user in different ways. For instance, using visual means, the force applied can be represented as lights, light patterns, or visual representation through many different ways as the skilled person may envisage. For example, a certain light indicator may be used to indicate the force applied is within a certain range. The force may be also represented as a bar, a circle or any other graphical representation of the force, which provides easy reading of the force applied. For instance a bar may be filled up when the force is increased. Haptic means may provide subtle vibrations according to the force applied, and different frequencies or intensities may be used to indicate the amount of force applied. Using the auditory means, a similar approach may be used using different sounds to indicate the force applied with the device (100) onto the specimen (200).

Advantageously, feedback means (40) allows the user to easily determine whether the pressure applied onto the specimen is appropriate or not. The objective of feedback means (40) is to give real-time and preferably constant information to the user of the pressure exerted so that they place the pressure in the optimal zone and maintain the force. In this scenario, any system that allows information to be given to the user can be valid.

Particularly in Figs. 6A to 6D, it can be seen that the feedback element (40) provides the force applied in the form of a bar or gauge to indicate the user of the medical device (100) about the force with which the device contacts the specimen (200). In some embodiment, the feedback element (40) may further provide the force value and units, or an arbitrary force values to help the user understand the force applied.

In a preferred embodiment of the first aspect of the invention the feedback means (40) are configured to further provide feedback to the user of the medical device (100) about whether the force is within the predetermined range of force or not. To do so, the feedback element may include certain ways of indicating through the feedback means the relative force applied with regard to the predetermined range of force. For example, using visual feedback means (40), a gauge can be used where the minimum and maximum values of the predetermined range of force are indicated and the force applied is represented as a bar, such that the user can determine whether to apply more or less force. Alternatively or additionally, a colour-scheme system can be used. For example, blue may indicate insufficient force, green optimal force and red high force. Using haptic feedback means, the vibration can be produced such that when the force is provided within the predetermined range of force, the vibrations stop, and when outside, they are produced, such to indicate to the suer that a change in force shall be applied.

Advantageously, this further allows the user to easily determine whether the pressure applied onto the specimen is appropriate or not and how to correct the pressure applied such that it is appropriate according to the predetermined range of force.

In Figs. 6A to 6D, it can be seen that the feedback element (40) provides feedback whether the pressure applied onto the specimen is appropriate or not and how to correct the pressure applied such that it is appropriate according to the predetermined range of force, the minimum and maximum values of the range indicated as two lines across the bar. If the force is between the two lines, the force is within the predetermined range of force.

As can be seen, Figs. 6A, 6B, 6C and 6D show a visual feedback device interface (40) before use (A), in use but before torsional waves are induced through the specimen (B), in use while torsional waves are induced through the specimen (C), and after torsional waves have been induced through the specimen (D). They include many other additional features, which may not be included in other embodiments or which may be understood in different ways. For example, they all further comprise messages to the user, but according to other feedback means (40) there may not be any message or may not be able to provide messages such as in haptic feedback means. Figs. 6B and 6C also show an image while in use but in some other embodiments this may not be the case.

In another preferred embodiment of the first aspect of the invention, and as shown in Fig. 5 the feedback means (40) are located at the proximal end (110) of the medical device (100). The proximal end (110) is the part of the medical device (100) closest to the user. This greatly facilitates the user to determine whether the pressure applied onto the specimen is appropriate or not while the medical device is in use, such that the focus of the user can be kept in the medical device (100) and the specimen (200).

In another particular embodiment of the first aspect of the invention, the visual feedback element (41) is configured to provide the measurements result after the medical device (100) has contacted the specimen (200) and the torsional ultrasonic waves transducer (10) has finished inducing the torsional wave through said specimen (200).

As can be seen, Fig. 5 shows the proximal end of a medical device according to one or more embodiments of the invention. It includes many other additional features, which may not be included in other embodiments or which may be understood in different ways. For example, the medical device (100) of Fig. 5 comprises a handle (50), but in other embodiments the proximal end (110) may not comprise a handle (50) or may comprise a handle (50) with a shape. The proximal end (110) also comprises a set of control buttons (112), but in other embodiments the proximal end (110) may not comprise two control buttons (112), it may comprise more, less or none control buttons (112). The control buttons (112) may also take different shapes, sizes and locations.

In another preferred embodiment of the first aspect of the invention, and as shown in Figs. 4, 5 and 6, the medical device further comprises an imaging means (41), wherein the feedback means (40) comprise a visual feedback device configured to display the images captured by the imaging means (41).

The imaging means (41) may be any type of means configured to capture an image of the medical device (100) as it contacts the specimen (200). The imaging means (41) may be for instance a camera or an ultrasound system such as an echographer. However, the skilled person may envisage other imaging means (41) that may also be used to view how the medical device (100) as it contacts the specimen (200), such as a lidar. The visual feedback device may be configured to display the images captured by the imaging means (41) continuously or on an ad hoc basis when necessary.

Fig. 4 shows a close up view of the distal end (110) of a medical device (100) according to one or more embodiments of the invention. It can be noticed how the distal end (110) comprises the imaging means (41) within the same area that comprises the torsional ultrasonic waves transducer (10) indicated through the rings of the receiver (14) around the contact element (11) as will be explained further below in a more particular embodiment. Nevertheless it is noted that given other torsional ultrasonic waves transducer (10), the imaging means (41) may be arranged differently in order to allows the user how it contacts the specimen (200).

It is noted the medical device (100) may further comprise lighting means associated to the imaging means (41) in order to facilitate or improve the capture of images from the specimen.

In an alternative embodiment, the imaging means are external to the medical device (100), such as an external echographer. Having visual feedback device as feedback means (40) allows for displaying the images captures by the imaging means (41), as can be seen in Figs. 6B and 6C. This in turn allows for the user to see how the medical device (100) is being contacted into the specimen (200). In many applications, such that of uterus cervix, it is desired to know how the torsional ultrasonic waves transducer (10) located at the distal end (190) of the medical device (100) contacts the cervix (200), as there is no direct vision of the cervix (200) for the user.

In a more preferred embodiment of the first aspect of the invention, and as shown in Figs. 3 and 4, the imaging means (41) are located at the distal end (190) of the medical device (100), and the imaging means (41) and the visual feedback device (40) are coaxially located within the medical device (100). Thus, the visual feedback device (40) can display the images captures by the imaging means (41) such that a movement of the imaging means (41) in a certain direction is also reflected as a movement in the same direction in the visual feedback device (40).

Advantageously, this allows for the user for an intuitive control of the medical device (100) such that the movements applied into the medical device (100), are directly translated into the exact movements seen through the visual feedback device (40).

In a more preferred embodiment of the first aspect of the invention, and as shown in Fig. 3 the imaging means (41) and the visual feedback device (40) are coaxially within the medical device (100) along the axis (130) formed by the distal end (190) and proximal end (110).

Advantageously, this further allows for the user for an intuitive control of the medical device (100) such that the movements applied into the medical device (100), are directly translated into the exact movements seen through the visual feedback device (40). For example, when the specimen is a uterus cervix (200) since the user of the medical device is not able to see how the distal end (190) of the medical device (100) contacts the cervix (200) it is very desirable that the imaging means (41) and the visual feedback device are coaxially within the medical device (100) along the axis (130) formed by the distal end (190) and proximal end (110), since the imaging means (41) is in the distal end, and the user looks at the visual feedback device (40) in the proximal end (110).

As can be seen, Fig. 3 shows the proximal end of a medical device according to one or more embodiments of the invention. It includes many other additional features, which may not be included in other embodiments or which may be understood in different ways. For example, the medical device (100) of Fig. 3 comprises a handle (50), but in other embodiments the proximal end (110) may not comprise a handle (50) or may comprise a handle (50) with a shape. It is also noted that the axis (130) is located with a certain angle (α) with respect to the handle (50), but in other embodiments, this angle (α) may be different.

In another particular embodiment of the first aspect of the invention, and as shown in Fig 1B, the force sensor (20) is comprised within the distal section (180) of the medical device (100).

Advantageously, this further reduces the noise introduced into the force sensor (20), therefore providing a more accurate reading of the force with which the device contacts the specimen (200). Since the force sensor captures the force applied to it, it is very desirable to reduce as much as possible the noise recorded by the force sensor. By placing the force sensor within the distal section (180) of the medical device (100), the forces applied to the force sensor are not damped by intermediate elements between the distal end (190) where the specimen (200) is contacted at, so the force sensor (20) increases its signal-to-noise ratio (SNR).

In another particular embodiment of the first aspect of the invention, and as shown in Fig 1B, the force sensor (20) is located perpendicularly to the surface of the medical device (100) configured to contact the specimen (200).

Advantageously, this further ensures the force sensor (20) only reads axial forces applied onto the specimen, which further reduces the noise introduced into the force sensor (20), such as transversal forces, therefore providing a more accurate reading of the force with which the device contacts the specimen (200).

It is important to note that the predetermined force ranges for the torsional ultrasonic waves transducer, it has been defined by only considering for the pressure of the emitter-receiver assembly on the specimen. In this case, no lateral forces are counted for, but only the force perpendicular to the specimen exerted by the axis of the tip of the device, where force-emitter-receiver systems are aligned.

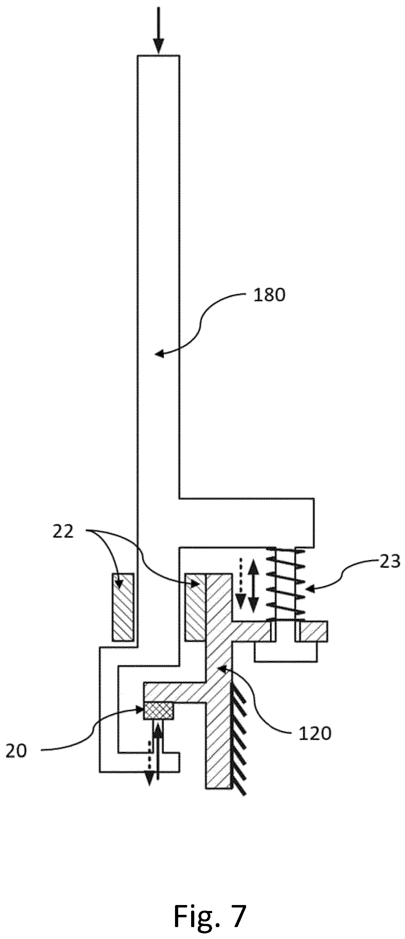

In another particular embodiment of the first aspect of the invention, and as shown in Fig. 7, the force sensor (20) is comprised between a proximal section (120) of the medical device (100) and a distal section (180) of the medical device (100). Advantageously, this means that when the specimen is contacted with the distal section (180) and the user applies the force through the proximal section (120), the transmitted force can be measured as the force sensor (20) is arranged between the two.

In an even more preferred embodiment, the force sensor (20) determines the force with which the device (100) contacts the specimen based on the relative position of the distal section (180) to the proximal section (120). Advantageously, this allows the sensor to be located between the proximal section (120) and the distal section (180), allowing the force sensor (20) to measure the relative position between the two sections derived from the force applied to the specimen. For example, the sensor may have an elastic element that deforms as pressure is applied, the deformation of which applies a force on the force sensor (20). Alternatively, the sensor can simply sit between the two sections and measure the force applied between them. The subject matter expert can envisage a number of other alternatives by which the force sensor (20) is able to measure the relative position between the proximal section (120) and the distal section (180). The force sensor (20) can, in accordance with this even more preferred embodiment, measure the change in the relative position between the proximal section (120) and the distal section (180) in various directions, including both the transverse and longitudinal relative position.

In an even more preferred embodiment of the previous embodiment as shown in Fig. 7, the force sensor (20) determines the force with which device (100) contacts the specimen when the distal section (180) moves longitudinally with respect to the proximal section (120). Fig. 7 shows a diagram of the force sensor mechanism (20) of a device (100) according to one or more embodiments of the invention. Advantageously, this embodiment allows the force exerted by the user of the device (100), which is transmitted longitudinally along the device (100), to be the force directly measured by the force sensor (20) through the longitudinal displacement of the proximal section (120) with respect to the distal section (180), preferably when the sections approach each other.

As shown in Fig. 7, the distal section (180) can be moved longitudinally with respect to the proximal section (120) when a force (F) is applied to the specimen through the distal section (180). When the distal section (180) is moved, the force sensor (20) located between the proximal section (120) and the distal section (180) can determine the force with which the device (100) contacts the specimen. For example, it can work by detecting the force exerted on it when both sections are longitudinally approached, or by detecting the release of force when the force sensor (20) is passively subjected to a given force and longitudinal displacement by bringing both sections closer together releases some of that passively applied force. The subject art expert can see a number of ways in which the force sensor (20) can determine the force with which the device (100) contacts the specimen when the distal section (180) moves longitudinally with respect to the proximal section (120).

Fig. 7 shows a diagram view of the operation of the force sensor mechanism (20) of a device (100) according to the present embodiment. However, the diagram includes many other additional features, which may not be covered in other embodiments or which may be comprehended in different ways.

For example, it can be seen that the distal section (180) and the proximal section (120) have a certain schematic shape that allows longitudinal displacement between them. Thus, the distal section is moved through a bearing (21) that allows longitudinal sliding between the two sections, minimizing friction and wear while guiding the displacement. However, in other embodiments, the distal section (180) could use another solution to guide the longitudinal displacement, or it could have no element guiding the longitudinal displacement. It can also be seen that the force sensor (20) in Fig. 7 determines the force with which the device (100) contacts the specimen when the distal section (180) is displaced longitudinally with respect to the proximal section (120), measuring the release of a passive force by this displacement. To this end, it comprises a number of optional additional elements that will be described below with respect to preferred embodiments.

In an even more preferred embodiment, and as described in the diagram in Fig. 7, the force sensor (20) is preloaded and determines the force with which the device (100) contacts the specimen according to the release of pressure on the specimen when the distal section (180) is displaced longitudinally with respect to the proximal section (120). Advantageously, this allows for a higher sensitivity in the force sensor (20) by defining the no-load reference force by default.

The force sensor (20) is pre-loaded in such a way that when device (100) is not in use, the sensor receives a constant force from another element (23). Preferably, this force is given by an elastic element (23) such as a spring, which applies a force either directly to the force sensor itself (20) or to another element that transmits it to the force sensor (20). Thus, in Fig. 7 it can be seen that device (100) comprises an elastic element (23) in the form of a spring, which exerts a force in such a way that both distal (190) and proximal (110) sections are passively separated. In addition, it can be seen that the distal section (180) has a proximal zone in itself, a clamp-like section in such a way that it surrounds the force sensor (20) and the proximal section has a support in contact with the force sensor (20) in such a way that the preload produced by the elastic element (23) generates a passive pressure on the force sensor (20). This occurs when the distal section (180) is separated from the proximal section (120), and the support of the proximal section (120) is in the path of the distal section (180) and the force sensor (20) is between the two, making a passive compressive force effect.

Once the device (100) comes into contact with the specimen and a force is applied to it, the passive force provided by the spring (23) is defeated, and the passive force applied to the force sensor (20) is released, a phenomenon that the force sensor (20) interprets as an externally applied force.

In another particular embodiment of the first aspect of the invention, and as shown in Fig 1B, the force sensor (20) further comprises a protection system configured to prevent the force sensor (20) from getting damaged when a force exceeding the working range is applied to the distal end (190) of the medical device (100).

Since the force sensor (20) is very sensitive in order to provide accurate measurements regarding the force with which the device (100) contacts the specimen (200), it may be exposed to damage if a strong force is applied to the distal end (180) of the device accidentally, for example by resting the device (100) on its distal end (190), or if it fall down.

Advantageously, this ensures that if the medical device is dropped, or an excessive force is applied onto the distal end (190) of the medical device (100), the force sensor (20) does not break.

To do so, the protection system may for example allow for a longitudinal displacement of the force sensor, such that the force provided is not absorbed exclusively by the force sensor, thus preventing the force sensor from breaking.

In a more preferred embodiment, as seen in Fig. 1B, the protection system comprises a spring (21). A spring can be tuned with an appropriate spring constant such that when certain force is applied it deforms.

In another particular embodiment of the first aspect of the invention, and as shown in Figs. 2 and 3, the medical device (100).

further comprises a handle (50) on its proximal end (110) configured to be held by a hand and with an angle (α) comprised between 85° and 120° with respect to the axis (130) formed by the distal end (190) and proximal end (110). In a preferred embodiment, the handle is configured with an angle between 85° and 115° with respect to the axis (130) formed by the distal end (190) and proximal end (110), more preferably between 90° and 110°.

Advantageously, an angle (α) comprised between 80° and 150°, preferably between 85° and 120° with respect to the axis (130) formed by the distal end (190) and proximal end (110) has been found to enable a significant increase in the dexterity and command of the medical device, further enabling a better control of the force exerted with the distal end (190) of the medical device (100) onto the specimen (200). This is because this angle range has been shown to enable the alignment of the natural movement of the arm and wrist and the movement of the medical device (100) towards the specimen (200). Thus, it allows for a simpler way for ensuring the device contacts the specimen (200) within the predetermined range of force.

In another particular embodiment of the first aspect of the invention, the handle (50) has a diameter between 30 and 150mm in any direction across its main axis.

In another particular embodiment of the first aspect of the invention, the handle (50) comprises a surface with a rugged material configured to increase the grip.

In another particular embodiment of the first aspect of the invention, the handle (50) comprises a indentations configured to conform to the fingers of the user.

In another particular embodiment of the first aspect of the invention, the processing unit (30) is configured to discard the readings of torsional ultrasonic waves transducer (10) if the force applied is not within the predetermined range of force.

Advantageously, this allows the medical device (100) able to ensure that only the reading within the predetermined range of force are used, which significantly increases the reliability of the measurements provided to the user. For example, the torsional ultrasonic waves transducer (10) may perform 10 readings each time the user requires a reading, while only a portion of them are used to determine the values of the torsional wave that goes through said specimen (200), as some are discarded as the force applied to the specimen (200) was to high or too low.

In another particular embodiment of the first aspect of the invention, the processing unit (30) is configured to prevent the torsional ultrasonic waves transducer (10) from inducing torsional waves if the force applied is not within the predetermined range of force.

Advantageously, this allows the medical device (100) able to ensure that the device does not start inducing torsional waves through the specimen (200) unless the force provided is within the predetermined range of force are used, which significantly increases the reliability of the measurements provided to the user.

For example, as shown in Figs 6A and 6B, which show that the force applied is below the minimum value within the desired range, the processing unit (30) is configured to prevent the torsional ultrasonic waves transducer (10) from inducing torsional waves.

Even when the user has indicated that it desires to induce torsional waves through the specimen (200), the processing unit can prevent this from happening unless the force applied is within the predetermined range of force of the torsional ultrasonic waves transducer (10) for the application of interest.

In another particular embodiment of the first aspect of the invention, the medical device (100) comprises a dummy section between the distal section (180) and proximal section (120), configured to increase the distance between the distal end (190) and the handle (50). Advantageously, this prevent the user from contacting the specimen (200) or any other sensitive element while the medical device (100) is being used. For example, when the medical device is a probe (100) configured to contact a uterus cervix (200), having a probe (100) with a dummy section that increases the distance between the distal end (190) and the handle (50) prevent the clinician from being obstructed by the patient's body or speculum, making the use of the probe (100) simpler and more user friendly for both the clinician and the patient.

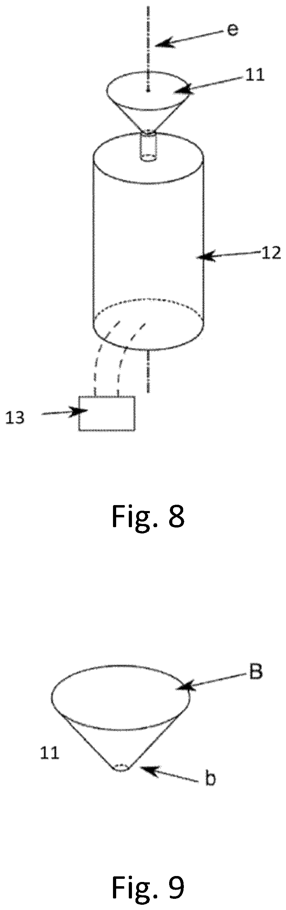

In another preferred embodiment of the invention, as shown with respect to Fig. 8, the torsional ultrasonic waves transducer (10) comprises an emitter device for emitting torsional ultrasonic waves, wherein the emitter device comprises an electrical signal generator (13) connected to an electromechanical actuator (12) which is in turn attached to a contact element (11) that comes into contact with a specimen (200), such that when the actuator receives electrical signals, it induces rotational movement of the contact element (11), and when said contact element (11) comes into contact with the specimen (200), it induces a torsional wave that goes through said specimen (200), wherein the torsional ultrasonic waves transducer (10) further comprises means for receiving the distorted signal after it goes through the specimen (200).

With this configuration, the wave transmitted by the transducer of the invention is a torsional wave, not a longitudinal wave, which improves the quality of the received signals. Unlike other known transducers having a flat wave front progressing in depth, the wave front achieved with the emitter of the invention is a radially-propagating and simultaneously-penetrating wave front (toroidal front).

Another aspect of the invention relates to the method for emitting torsional waves using the emitter of the invention.

In a particular embodiment, the electrical signal used for stimulating the actuator in this method will be an oscillating signal, more preferably a sinusoidal signal and even more preferably a sine signal.

In this case, the change in voltage over time corresponds to the following function: $V \left(t\right) = A ⋅ sin \left(ωt\right)$ where A is the maximum amplitude of the wave, corresponding with the maximum generating voltage.

In another particular embodiment, the contact element has a considerably frustoconical shape (Figure 9), such that its smaller base (b) is attached to the electromechanical actuator and its larger base (B) is arranged at the distal end of the transducer of the invention so that it comes into contact with the specimen on which shear wave is to be transmitted.

In a preferred embodiment, the contact element is made from a biocompatible material.

In another particular embodiment, the electromechanical actuator is covered by a Faraday cage which eliminates electronic noise. Specifically, the electromechanical actuator is wrapped with a conductive covering acting as a Faraday cage.

In another preferred aspect of the invention, the transducer is capable of generating a torsional ultrasonic pulse which propagates by going through the specimen, and it is capable of picking up the distorted pulse after it goes through the specimen. Said transducer ("transducer of the invention") is a transducer comprising the emitter of the invention and means for receiving the distorted signal after it goes through the specimen, hereinafter "receiver."

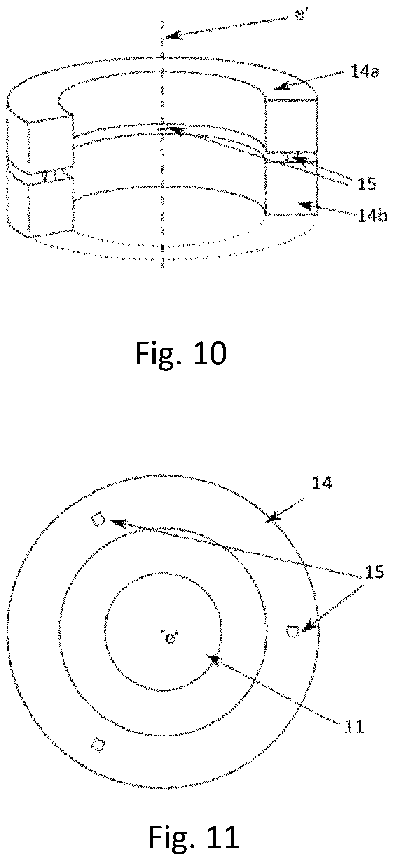

In a particular embodiment (Figure 10), the receiver of the transducer of the invention comprises two or more piezoelectric elements (15) located equidistant from one another and placed between two rings (121A and 121B). The rings are preferably made from a non-conducting material, more preferably a biocompatible material, such that each piezoelectric element is in contact with two electrodes of different charges, arranged perpendicular to the polarization of said piezoelectric elements.

In their preferred arrangement, (Figure 11), the axis of rotation (e') of the rings (14) of the receiver and the axis (e) of the contact element (11) must coincide with one another, the emitter being located inside the rings.

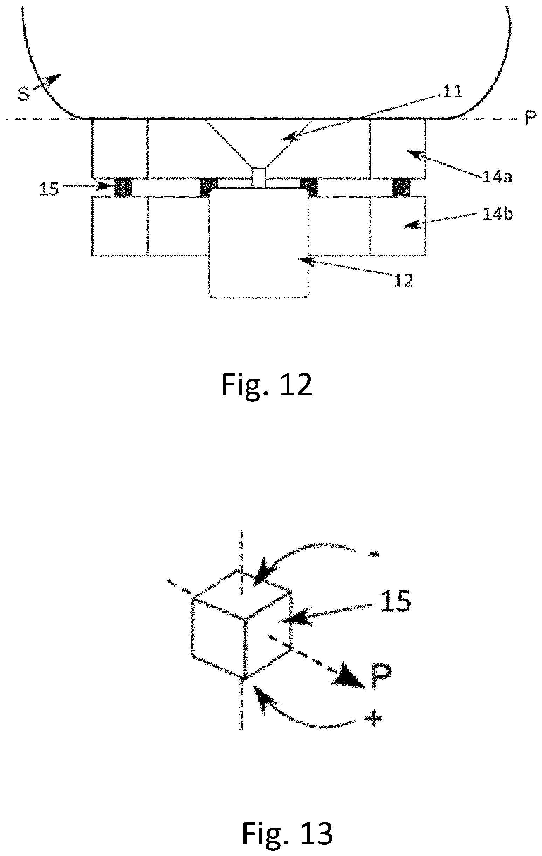

Likewise (Figure 12), in order for both the emitter and the receiver to be in contact with the specimen (S), the outer face of one of the rings, called the front ring (121A), and the flat surface of the contact element (11) must be located on the same plane (P) (plane of contact).

In another particular embodiment, the faces of the rings coming into contact with the piezoelectric elements (inner faces) will have their respective positive and negative poles connected, such that each ring will act independently as the anode and cathode.

Polarization, which is understood to be the direction between the positive and negative charges of the electrode, of the piezoelectric elements can be carried out in two different ways. In a preferred embodiment, the polarization is parallel to the axis, the electrodes being arranged on side faces of said piezoelectric elements; in a more preferred embodiment, the polarization (P) is perpendicular to the axis in the radial direction, the electrodes being arranged in the attachment between said piezoelectric elements and the rings (Figure 13).

In a preferred embodiment, the piezoelectric elements (15) are made from piezoelectric ceramic PZT-4 or PZT-5.

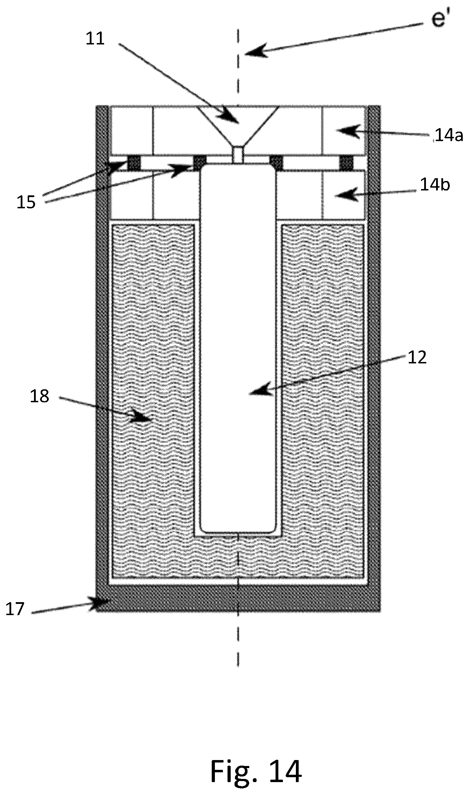

The transmission and reception elements of the transducer are arranged inside a casing (7, Figure 14) which, in addition to protecting the transducer against physical impacts (such as falls or scratches), assures device functionality as each element is fixed in its correct position.

In the particular case in which the receiver of the transducer of the invention is formed by concentric rings, the casing must allow the emitter to remain located inside said rings, such that the axes of rotation thereof.

In a preferred embodiment, the casing is made from polylactic acid (PLA).

Optionally, in another particular embodiment the transducer of the invention further comprises an attenuating element (18), preferably an attenuating material with a shore A lower than 80, fixed to the outer face of the ring that is the farthest away from the area of contact with the specimen, in order to prevent the propagation of torsional waves in the direction opposite that of the specimen, and therefore also preventing energy losses. The effective emission of torsional waves thereby occurs on only one face of the transducer, i.e., the face put in contact with the specimen, the oscillation of the rear face being cancelled by means of the attenuating element. Furthermore, the cancellation of the emitted waves in the direction opposite the direction of the specimen means that the emitted waves require a simpler processing, as a cleaner signal is achieved.

In an even more particular embodiment, the transducer of the invention which allows emitting and receiving torsional waves comprises the following elements:
- An emitter comprising:
   ∘ an electrical signal generator
   ∘ an electromechanical actuator connected to the electrical signal generator and covered by a Faraday cage,
   ∘ a contact element attached to the electromechanical actuator such that when the actuator receives electrical signals, it induces rotational movement of the contact element; and
- A receiver comprising:
   ∘ two rings preferably made from a non-conducting material,
   ∘ two or more piezoelectric elements arranged between the preceding rings and separated equidistantly.
- A casing which allows the emitter to remain located inside the receiver such that the axes of the contact element and the rings coincide with one another and the outer part of said contact element and the outer face of one of the rings remain on the same plane, such that they can come into contact with the specimen.

Furthermore, in another more preferred embodiment the transducer is completed with a latex membrane adapted to the shape of the device, assuring the dissipation of the wave travelling therethrough with an adapted involution between the emitter and the receiver.

### Second aspect of the invention

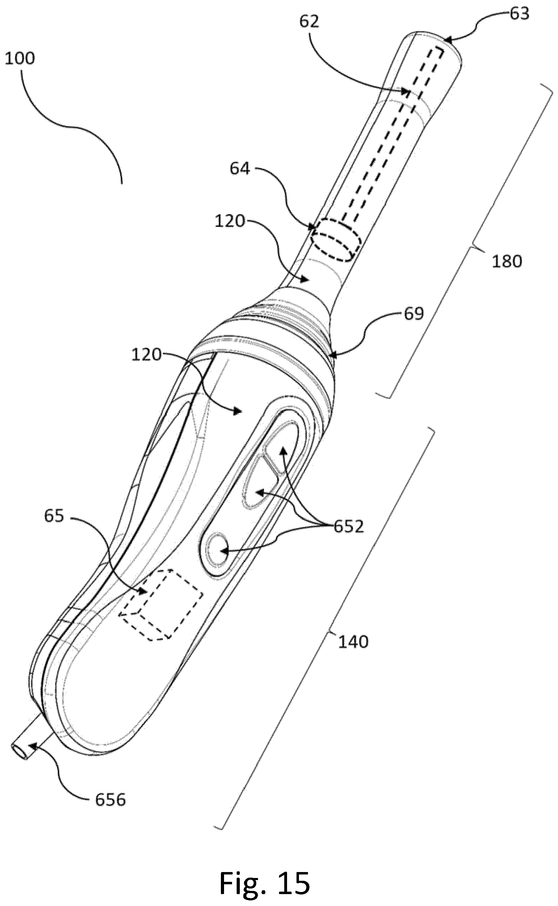

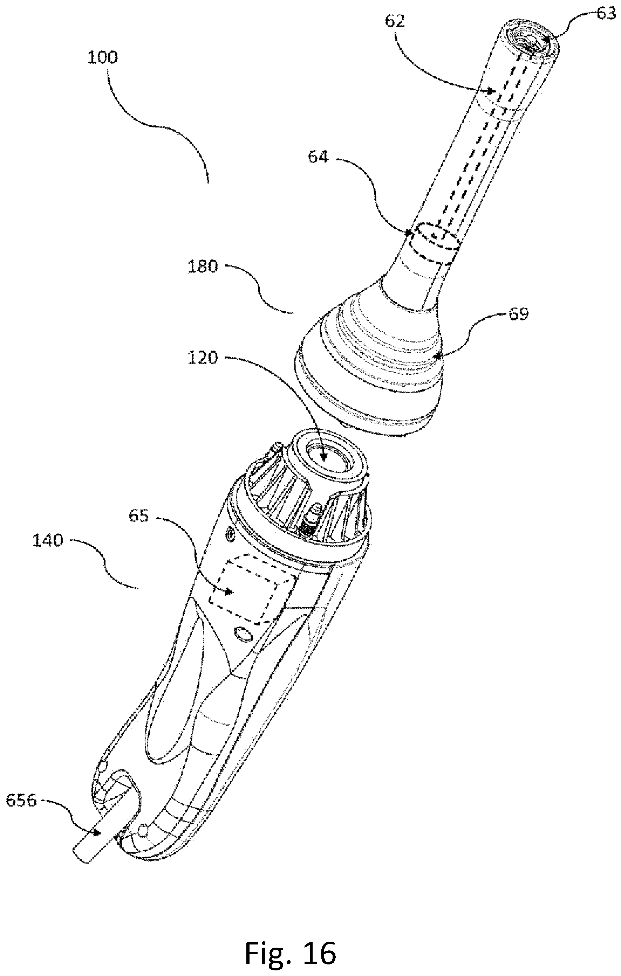

A second aspect of the invention as shown in Figs. 15 and 16, concerns a medical device (100). It is noted that the medical device (100) of the second aspect of the invention may be the same medical device of the first aspect of the invention or a different medical device. It is therefore understood that any of the embodiments of the first aspect of the invention may comprise features according to any of the second aspect of the invention in any combination possible and vice versa.

The medical device (100) comprises a proximal section (120) and a distal section (180) and is configured to contact a specimen. Figs. 15 and 16 show a perspective view of a medical device (100) according to one or more embodiments of the invention, Fig. 16 showing the distal (190) and proximal (110) sections in exploded view according to one or more embodiments of the invention. For economy of language, the term "device" will henceforth be used in reference to a medical device.

It is noted that the proximal section (120) and distal section (180) of the device (100) refer to distinct portions of the device (100) that may or may not be defined to the same length. For example, in some embodiments, the proximal section (120) may comprise the majority of the length of the device (100) while the distal section (180) comprises the remaining minority of the device (100); and vice versa. In addition, as shown in Fig. 16, the proximal section (120) and the distal section (180) may be physically separated into two separate parts, however in other embodiments, the proximal (110) and distal (190) sections may not be physically separated but may be limited to different sections of the device (100) although they belong to the same piece of the device. Thus, in some embodiments, the proximal (110) and distal (190) sections may simply be spatial concepts defined on the same solid body. In addition, Fig. 16 shows that the proximal (110) and distal (190) sections are mechanically joined by a threaded mechanism, for example, however, as the expert in the field can see, there are several valid alternatives to join both pieces. For example, you can use a clip mechanism, a closure through a screw that goes through both pieces, or a magnet system. These and the rest of the obvious alternatives for the expert in the field are also part of the present invention. In addition, it is observed that the device (100) has an essentially elongated shape, but in other embodiments it can take other non-elongated shapes.

According to the second aspect of the invention, the device (100) is characterized by a conduit (62), a sealing element (64) and a vacuum pump (65).

Conduit (62) is configured to connect the outside to the inner space (12) of the device (100), so that conduit (62) is connected to the outside through an opening (63). Conduit (62) may be a tube configured to make the connection between the interior space (12) and the exterior, in such a way that the tube terminates at the opening (63), or it may be a cavity of the device (100) in such a way that a connection between the exterior and the interior space (12) of the device (100) is generated through such an opening (63). Therefore, it can be observed that the conduit (62) when it is a tube can have different shapes and be more or less regular, being able to take different shapes and widths along it. It can also have different lengths, including the possibility that the conduit (62) is almost symbolic, when the interior space (12) of the device (100) is accessed through the opening (63). In Figs. 15 and 16, conduit (62) connects the outside to the inner space (12) of the device (100) through the opening (63). It can be seen that the interior space (12) is defined in this case as the space of the device (100) that is not directly communicated with the outside through this opening (63), preferably comprising the vacuum pump (65). In the case of Figs. 15 and 16, it includes the entire proximal section (120) and part of the distal section (180), because although both sections are removably connected, in their use they define a single watertight space that includes all the electronics for the operation of the medical device (100).

In addition, it is also observed that the aperture (63) can take different forms. For example, in some embodiments according to the present invention, the opening (63) may be a circular hole defined on the surface of the device (100), but in other embodiments the opening (63) may be a longitudinal hole defined along a larger surface. It should also be noted that in some embodiments the device (100) may comprise two or more openings (63) defined on the surface of the device (100). In addition, when device (100) comprises more than one opening (63), the openings (63) may be the same as each other, or have different shapes and sizes. It is also possible that the openings are located in different locations on the surface of the device (100).

The sealing element (64) is located at the opposite end of the conduit (62) to the opening (63). In this way, the sealing element (64) is configured to control the passage of air between the interior space (12) of the device (100) and the conduit (62), so that all fluid communication is made through the sealing element (64). The sealing element (64) must therefore cover the entire body of the device between the conduit (62) and the interior space (12) of the device (100), and be effectively adapted to its internal shapes. The sealing element (64) may consist of one or more sealing elements, such as a single piece of rubber, located between the two spaces, or be a plastic part forming part of the same device (100). In Fig. 15 it is shown as a cylindrical piece covering the entire width of the device body (100) between the conduit (62) and the interior space (12) of the device (100).

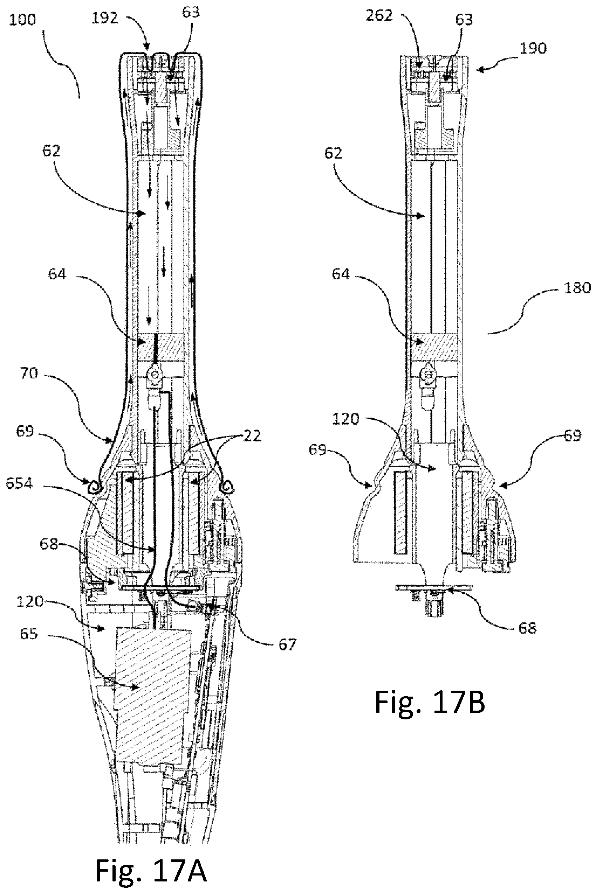

The vacuum pump (65) is schematically depicted in Figs. 15 and 16 and is connected to the sealing element (64) in such a way that it is configured to extract air from the opening (63) through the conduit (62). Therefore, the sealing element (64) not only controls the passage of air between the interior space (12) and the conduit (62), but also ensures that this passage of this air is controlled by the vacuum pump (65) connected to the sealing element (64). For such a connection, the vacuum pump (65) can either be integrated into the sealing element (64) or connected via a series of fluid connections such as a tube (54) as described below with reference to Fig. 17A. The vacuum pump (65) can be of various types, e.g. rotary vane, diaphragm or diaphragm or side channel, among others. Therefore, the vacuum pump (65) of the present invention is not limited to any particular type of vacuum pump (65).

Device (100) is configured to receive a membrane (70) on its outside covering the opening (63). Device (100) can be configured to receive a membrane (70) in a variety of ways. For example, having a series of fixings for the membrane (70) that allows the membrane (70) to be fixed to the device (100). For example, fasteners can be in the form of a hook or indentation, where the membrane (70) can be fixed. Alternatively, device (100) may have a characteristic shape that allows a membrane (70) with a specific and complementary shape to be fitted over it.

As the device is configured to receive a membrane (70) on the outside covering the opening (63), this implies that the vacuum pump (65) is configured to extract the air between the sealing element (64) and the membrane (70). This is because the sealing element (64) controls the air passage between the interior space (12) and the conduit (62) governed by the vacuum pump (65); and since the conduit (62) communicates with the outside through the opening (63), being at the opening (63) covered by the membrane (70), the vacuum pump (65) is configured to extract the air between the sealing element (64) and the membrane (70), as shown in Fig. 17A.

Fig. 17A shows a sample of a longitudinal section of a device (100) according to one or more embodiments of the invention. As can be seen, device (100) is covered by a membrane (70) covering the opening (63). In addition, as shown by the directional arrows, the air between the sealing element (64) and the membrane (70) is extracted by means of the vacuum generated by the vacuum pump (65) at the level of the sealing element (64), which allows the correct adjustment of the membrane (70) on the device (100).

In addition, as mentioned above, in some projects the vacuum pump (65) can be connected to the sealing element (64) via a series of fluid connections (54), such as a vacuum tube, which can also be extended through the sealing element (64) itself to the conduit (62).

Advantageously, device (100) of the present invention allows the membrane (70) that is placed on it to be adjusted to the surface of the device (100) quickly and efficiently, in such a way as to ensure a correct arrangement on it in such a way that the functions of the medical device (100) are not altered.

As shown in Fig. 15, in some implementations, the vacuum pump (65) can be controlled by the user of the medical device (100) via a series of interfaces (652) on the device itself (100), such as buttons or external controls. In this way, the user can control the switching on and off of the vacuum pump (65) and/or its level of negative pressure exerted. The subject matter expert may appreciate other controls to include, such as choosing from a series of preset programs or functioning as a safety system so that the pump only works when a combination of interfaces (652) are pressed simultaneously. In other embodiments, the vacuum pump (65) can be configured to generate the vacuum continuously without the need for interaction from the user of the device (100), and therefore the interfaces (652) would not be present. In other embodiments, device (100) can include a sensor that lets it know when the membrane (70) is placed on the device (100), and activate the vacuum pump (65) only when a membrane (70) is arranged on the device (100). In other versions, the vacuum pump (65) can be controlled remotely by another control device. For this purpose, interfaces (652) are optional.

For the control of the vacuum pump (65) the device (100) may optionally comprise a processor configured to control the operation of the vacuum pump (65) and/or a memory unit configured to store the instructions to be executed by the processor. It is emphasized that the processor can comprise one or more processing units, such as a microprocessor, GPU, CPU, multi-core processor, or similar. Similarly, memory may comprise one or more volatile or non-volatile memory devices, such as DRAM, SRAM, flash memory, read-only memory, ferroelectric RAM, hard disk drives, floppy disks, magnetic tapes, optical discs, or the like. The driver may be implemented in software (a computer program), hardware (a physical device), or any combination in order to execute the sequences of operation disclosed herein.

It can be seen that the vacuum pump (65) can be supplied externally by a series of power cables (656) as shown in Figs. 15 and 16, but in other embodiments, the vacuum pump (65) can be powered by internal batteries or internal power.

As can be seen, Figs. 15, 16 and 17A show a medical device (100) that includes many other additional features, which may not be included in other embodiments or which may be understood in different ways.

For example, in Figs. 15 and 16 it can be seen that device (100) has an essentially elongated shape, but other devices (100) according to this invention may not be essentially elongated. It can also be seen that the distal section (180) is narrower than the proximal section (120), and that the proximal section (120) comprises almost all the electronics of the device (100). However, in other embodiments, the device may comprise other shapes, and the proximal (110) and distal (190) sections have a similar, same, or different shape depending on the application of the device itself (100). In this sense, the present invention is not limited to any particular form of the device (100). In both figures it can be seen that the device (100) comprises a power cable (56), however in other embodiments the cable may not be there, and the device may be powered by an internal power source such as a battery or it may comprise two or more cables (656) as an information cable that allows it to be electronically connected to another device. It can also be seen in Fig. 15 that device (100) comprises three interfaces (652) configured to control it. However, in other embodiments, device (100) may comprise more or fewer interfaces (652) or may not comprise any interfaces (652) as described above. In Fig. 16 it can be seen that the opening (63) is located at the distal end of the distal section (180) of the device (100) and that it has a circular shape, however in other embodiments, the opening (63) can be found in another location and have other shapes and sizes, or have several openings (63) as described above.

As for Fig. 17A, as mentioned above, it can be seen that the vacuum pump (65) is located in the proximal section (120) and connected to the sealing element (64) through a tube (54), however in other embodiments the vacuum pump (65) may be located next to the sealing element (64) or included in the sealing element itself (64). It can also be seen that the membrane (70) is arranged in a particular shape over the distal section (180) of the device (100) with a particular distal end. However, in other embodiments, device (100) may take other forms. In addition, a number of optional additional elements can be appreciated, which will be described below with respect to preferred embodiments.

In a preferred embodiment, as shown in Fig. 17A, the vacuum pump (65) is connected to a pressure sensor (67) configured to measure the pressure in the conduit (62). The pressure sensor (67) can be of various types, for example a piezoresistive, capacitive, electromagnetic, piezoelectric, strain gauge, optical or potentiometric gauges, among others. Therefore, the pressure sensor (67) of the present invention is not limited to any particular type of pressure sensor.

In Fig. 17A, the pressure sensor (67) is connected to the vacuum pump (65) via a fluid connection (57) that fluidly connects the pressure sensor (67) to the tube (54) which in turn is connected to the vacuum pump (65) and the conduit (62). However, in other versions, the pressure sensor (67) may be included in the vacuum pump (65) or connected to the conduit (62), the vacuum pump (65), the tube (54) or the sealing element (64). The subject matter expert can foresee the different ways in which a pressure sensor (67) can be positioned in such a way that it can measure pressure exerted in the conduit (62).

In addition, in this implementation, the vacuum pump (65) is configured to adjust its operation depending on the pressure measured by the pressure sensor (7). Depending on the pressure sensor (7), the vacuum pump (65) can be adjusted or not. Preferably, the vacuum pump (65) is configured to maintain a minimum pressure in the space between the membrane (70) and the sealing element (64). This can be done in a variety of ways, for example, the vacuum pump (65) can be configured to maintain a negative pressure relative to the given ambient pressure, so that it activates until this value is reached. Once the pressure rises above this value or a different value indicating that there has been a loss of vacuum, the pump can be turned on again until the pressure is reached again. Alternatively, the pump can be continuously activated and its vacuum power can be modified in such a way that , and when the minimum pressure is reached, its drain level is reduced, and when the pressure rises above that value or a different value indicating that there has been a loss of vacuum, the drain pressure increases again until the minimum reference pressure value is reached. To this end, in those devices that comprise it, the memory of the device (100) can store the instructions corresponding to that algorithm and the processor of the device (100) can execute these instructions stored in the memory. Alternatively, the memory and/or processor may be externally comprised and the vacuum pump (65) control may be directed from the outside.

Advantageously, this preferred embodiment allows the device (100) to ensure that the vacuum pump (65) maintains pressure over time, so that the membrane (70) is adjusted at all times. This is especially useful when device (100) is used on a specimen that may cause the membrane (70) to be displaced or part of the vacuum to be lost, increasing the pressure in the space between the membrane (70) and the sealing element (64).

In some embodiments, where device (100) comprises one or more interfaces (52), the interfaces (652) can set the target pressure of the vacuum pump (65) in such a way that the minimum pressure to be maintained can be regulated. In addition, the device (100) may comprise a visual interface, such as a display or a series of LED lights that allows the user (100) to have an awareness of the minimum set pressure that the vacuum pump (65) is configured to maintain. Advantageously, this allows the user of the device (100) to be able to adjust the minimum pressure in the space between the membrane (70) and the sealing element (64) during the use of the device (100), for example if conditions change.

In another preferred embodiment, device (100) is essentially elongated, and configured to contact the specimen through the distal section (180), and where device (100) is configured so that membrane (70) covers at least in the distal section (180). As shown in Figs. 1, 2 and 20A, the device (100) is essentially elongated, especially in its distal section (180). In addition, as shown in Fig. 17A, device (100) is configured so that membrane (70) covers at least the distal section (180) and since device (100) is configured to contact the specimen through the distal section (180), this implies that device (100) is configured to contact the specimen through membrane (70) which is tightly arranged by the vacuum pump (65). The opening (63) can be located in either the distal section (180) or the proximal section (120) where the membrane (70) is arranged, in those cases where the device (100) is configured so that the membrane (70) covers part of the proximal section (120).

In a more preferred embodiment, the opening (63) is included in the distal section (180) of the device (100). Advantageously, this allows that, since the membrane (70) is arranged on the distal section (180) and the device (100) is essentially elongated, the vacuum is generated from a point that allows the arrangement of the membrane (70) to be adjusted uniformly, thus avoiding sheets. Most preferably, the opening (63) is located at the distal end of the distal section (180). Advantageously, this allows for greater uniformity in the tight arrangement of the membrane (70), In those embodiments where the device (100) comprises more than one opening (63), according to the present preferred embodiment, the device (100) comprises at least one of the openings (63) in the distal section (180) of the device (100), preferably at the distal end of the distal section (180).

In another preferred embodiment, as shown in Fig. 17B, device (100) comprises a force sensor (20) configured to determine the force with which device (100) contacts the specimen. Fig. 17B shows a simplified view of the longitudinal section of the distal section (180) of a device (100) according to one or more embodiments of the invention.

As shown in Fig. 17B, device (100) comprises a force sensor (20) that is capable of recording the force with which the user contacts the device (100) with the specimen. Advantageously, this allows that in those embodiments where the pressure applied to the specimen affects the measurement by the device (100), the device (100) is able to measure that force. The force sensor (20) can be of many types and can be similar or the same as the pressure sensor (67) or not, for example a piezoresistive, capacitive, electromagnetic, piezoelectric, strain gauge, optical or potentiometric extensometer, among others. Therefore, the force sensor (20) of the present invention is not limited to any particular type of force sensor.

Preferably, the device comprises an interface (652) that allows the user to receive information about the pressure exerted on the specimen with the device (100) in such a way that it can be adjusted accordingly. The interface can comprise a display or a group of LED lights by defining a default code. tags. To this end, in those devices (100) that comprise it, the memory of the device (100) can store the instructions corresponding to that algorithm and the processor of the device (100) can execute these instructions stored in the memory. Alternatively, the memory and/or processor may be externally comprised and the information to be displayed to the user may be processed externally.

As with Fig. 17A, Fig. 17B shows a device (100) comprising many other additional features, which may not be included in other embodiments or which may be comprehended in different ways. Those in common, such as device (100) shape and sealing element (64) can be applied in the same way. It should also be noted that device (100) comprises the force sensor (20) at the proximal end of the distal section (180) of device (100). However, in other embodiments, the force sensor (20) may be included in other locations of the device (100) and have another type of mechanism of action.

In a more preferred embodiment, the distal section (180) is configured to contact the specimen, and the force sensor (20) is arranged between the distal section (180) and the proximal section (120). Advantageously, this means that when the specimen is contacted with the distal section (180) and the user applies the force through the proximal section (120), the transmitted force can be measured as the force sensor (20) is arranged between the two.

In an even more preferred embodiment, the force sensor (20) determines the force with which the device (100) contacts the specimen based on the relative position of the distal section (180) to the proximal section (120). Advantageously, this allows the sensor to be located between the proximal section (120) and the distal section (180), allowing the force sensor (20) to measure the relative position between the two sections derived from the force applied to the specimen. For example, the sensor may have an elastic element that deforms as pressure is applied, the deformation of which applies a force on the force sensor (20). Alternatively, the sensor can simply sit between the two sections and measure the force applied between them. The subject matter expert can envisage a number of other alternatives by which the force sensor (20) is able to measure the relative position between the proximal section (120) and the distal section (180). The force sensor (20) can, in accordance with this even more preferred embodiment, measure the change in the relative position between the proximal section (120) and the distal section (180) in various directions, including both the transverse and longitudinal relative position.

In an even more preferred embodiment of the previous embodiment as shown in Fig. 7, the force sensor (20) determines the force with which device (100) contacts the specimen when the distal section (180) moves longitudinally with respect to the proximal section (120). Fig. 7 shows a diagram of the force sensor mechanism (20) of a device (100) according to one or more embodiments of the invention. Advantageously, this embodiment allows the force exerted by the user of the device (100), which is transmitted longitudinally along the device (100), to be the force directly measured by the force sensor (20) through the longitudinal displacement of the proximal section (120) with respect to the distal section (180), preferably when the sections approach each other.

As shown in Fig. 7, the distal section (180) can be moved longitudinally with respect to the proximal section (120) when a force (F) is applied to the specimen through the distal section (180). When the distal section (180) is moved, the force sensor (20) located between the proximal section (120) and the distal section (180) can determine the force with which the device (100) contacts the specimen. For example, it can work by detecting the force exerted on it when both sections are longitudinally approached, or by detecting the release of force when the force sensor (20) is passively subjected to a given force and longitudinal displacement by bringing both sections closer together releases some of that passively applied force. The subject art expert can see a number of ways in which the force sensor (20) can determine the force with which the device (100) contacts the specimen when the distal section (180) moves longitudinally with respect to the proximal section (120).

Fig. 7 shows a diagram view of the operation of the force sensor mechanism (20) of a device (100) according to the present embodiment. However, the diagram includes many other additional features, which may not be covered in other embodiments or which may be comprehended in different ways.

For example, it can be seen that the distal section (180) and the proximal section (120) have a certain schematic shape that allows longitudinal displacement between them. Thus, the distal section is moved through a bearing (21) that allows longitudinal sliding between the two sections, minimizing friction and wear while guiding the displacement. However, in other embodiments, the distal section (180) could use another solution to guide the longitudinal displacement, or it could have no element guiding the longitudinal displacement. It can also be seen that the force sensor (20) in Fig. 7 determines the force with which the device (100) contacts the specimen when the distal section (180) is displaced longitudinally with respect to the proximal section (120), measuring the release of a passive force by this displacement. To this end, it comprises a number of optional additional elements that will be described below with respect to preferred embodiments.

In an even more preferred embodiment, and as described in the diagram in Fig. 7, the force sensor (20) is preloaded and determines the force with which the device (100) contacts the specimen according to the release of pressure on the specimen when the distal section (180) is displaced longitudinally with respect to the proximal section (120). Advantageously, this allows for a higher sensitivity in the force sensor (20) by defining the no-load reference force by default.

The force sensor (20) is pre-loaded in such a way that when device (100) is not in use, the sensor receives a constant force from another element (23). Preferably, this force is given by an elastic element (23) such as a spring, which applies a force either directly to the force sensor itself (20) or to another element that transmits it to the force sensor (20). Thus, in Fig. 7 it can be seen that device (100) comprises an elastic element (23) in the form of a spring, which exerts a force in such a way that both distal (190) and proximal (110) sections are passively separated. In addition, it can be seen that the distal section (180) has a proximal zone in itself, a clamp-like section in such a way that it surrounds the force sensor (20) and the proximal section has a support in contact with the force sensor (20) in such a way that the preload produced by the elastic element (23) generates a passive pressure on the force sensor (20). This occurs when the distal section (180) is separated from the proximal section (120), and the support of the proximal section (120) is in the path of the distal section (180) and the force sensor (20) is between the two, making a passive compressive force effect.

Once the device (100) comes into contact with the specimen and a force is applied to it, the passive force provided by the spring (23) is defeated, and the passive force applied to the force sensor (20) is released, a phenomenon that the force sensor (20) interprets as an externally applied force.

In another preferred embodiment of the invention, as shown in Fig. 17A, the membrane (70) is a prophylactic. Advantageously, this allows device (100) to be covered by a membrane (70) at a low cost and easy to obtain. Preferably, the prophylactic (70)additionally comprises a lubricant on its outer surface, facilitating the introduction of the device (100) through a conduit or cavity of the specimen.

In another preferred embodiment of the invention, device (100) comprises at least one indentation (69) in its outer surface configured to receive the edges of the membrane (70).

The indentation (69) can be formed in a variety of ways, as an expert in the field can appreciate. For example, the indentation (69) may form as a groove on the outer surface of the device (100), or it may be formed by arranging one or more radial extensions of the surface in such a way that an indentation is formed on the proximal surface of the device (100) radially. Very preferably, the indentation (69) will define a plane perpendicular to the longitudinal axis of the device (100). The indentation (69) can be defined at different heights of the device (100), both in the proximal section (120) and in the distal section (180). In a preferred embodiment, the indentation (69) will be defined at the proximal end of the distal section (180).

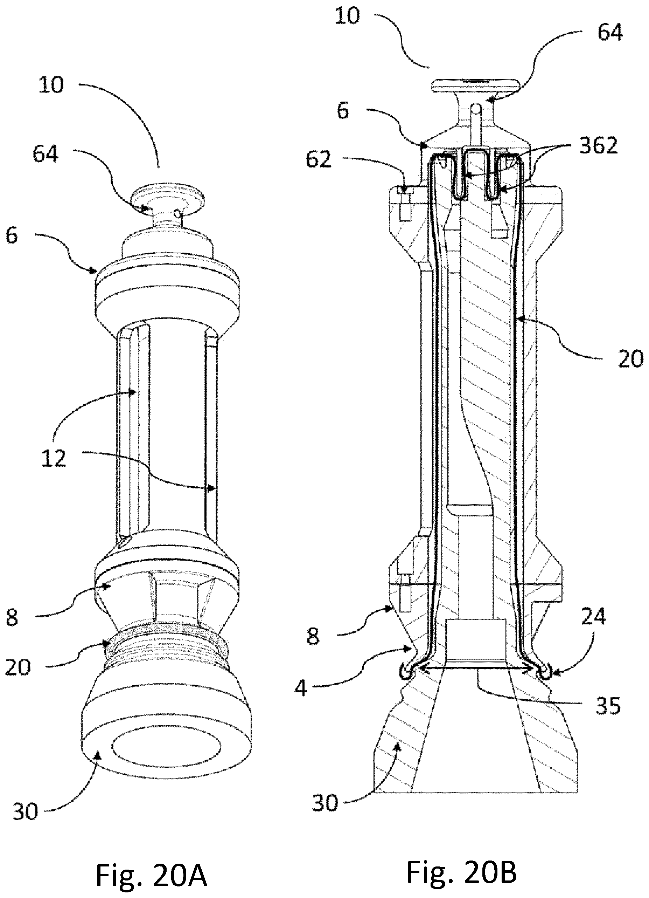

In addition, as can be seen in Figs. 20A and 20B, the indentation (69) is configured to receive the edge (69) of a membrane (70) covering the device (100). The membrane (70) is essentially elongated, preferably essentially cylindrical in shape, such that the edge (69) of the membrane (70) may comprise part of the membrane (70).

Advantageously, this design of the indentation (69) allows the device (100) to be configured so that once the membrane (70) is arranged in such a way that the edge (69) of the membrane (70) is received by the indentation (69), the membrane (70) is fixed in the device (100).

In an even more preferred embodiment, the indentation (69) is configured to receive the edges of the membrane (70) in a sealed manner. For example, the indentation (69) may contain a series of seals with a high coefficient of friction that generate greater tightness between the surface of the device (100) or it may be optimally designed to receive the edges (69) of the membrane (70). Advantageously, once the vacuum pump (65) has generated the minimum pressure, the surface of the membrane (70) is sealed in such a way that no air leaks can enter the defined space between the sealing element (64) and the membrane (70), resulting in a more efficient vacuum system.

In another preferred embodiment, device (100) is a probe. In this embodiment, device (100) is essentially elongated and is advantageously benefited by the solution proposed in the present invention, since it contacts the specimen with a distal section (180) where the membrane (70) is arranged, and can easily accommodate the opening (63) at the distal end of the distal section (180) since the probes in many cases include various solutions such as sensors or other surgical instruments at the distal end of its distal section (180). Advantageously, this more preferred method allows the same probe (100) to be used in several scans by having a mechanism to arrange the membrane (70) tightly over the probe (100) in several scans, simply by replacing the membrane (70) that is placed on the probe (100) in each of the scans performed.

In a more preferred embodiment, the device (100) is a uterine probe and the specimen is the cervix of a uterus. Preferably, the distal end (190) of the distal section (180) is configured to receive the cervix from a patient's uterus, using a design comprising an indentation (192) that allows the uterine cervix to be contacted once the probe is inserted into the vagina. It should be noted that the probe (100) in Figs. 1, 2, 20A, and 20B is merely representative, and that the uterine probe (100) can take different shapes, so that it is able to interact by different means, for different applications and/or with different orientations. Advantageously, this more preferred method allows the same uterine probe (100) to be used in different examinations in the same or different patients, simply by replacing the membrane (70) that is placed on the probe (100) in each of the examinations performed.

In an even more preferred embodiment, device (100) is a uterine torsional wave probe (100), comprising at its distal end (190)a torsional wave emitter and receiver. Preferably, the uterine torsional wave probe (100) is configured to characterize the structure of the uterine cervix. Advantageously, this allows the use of the same uterine torsional wave probe (100) in several examinations in the same or different patients, simply by replacing the membrane (70) that is placed on the probe (100) in each of the examinations performed. Even more preferably, the uterine torsional wave probe (100) is a torsional ultrasonic wave (100) probe.

Even more preferably, device (100) is a uterine torsional wave probe (100), such that it comprises a torsional wave emitting device which in turn comprises an electrical signal generator connected to an electromechanical actuator which in turn is attached to the distal end of the probe, so that when the actuator receives electrical signals, It induces a rotational motion of the contact element and this, when it comes into contact with the specimen, induces a torsion wave that passes through the specimen. Preferably, the probe (100) also includes means of receiving the distorted signal after it passes through the specimen. For example, media may comprise two or more piezoelectric elements located equidistant from each other and placed between two rings made of a non-conductive material.

### Third aspect of the invention

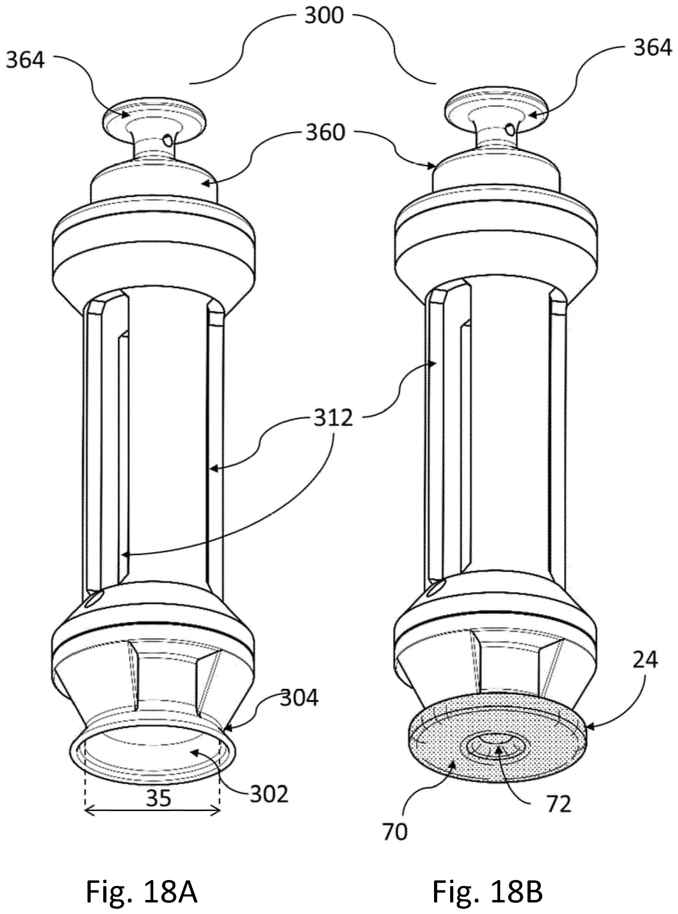

A third aspect of the invention can be seen in Figs. 18A and 18B, which show a perspective view of a device (300) configured for the arrangement of a membrane (70) in a probe (not shown) according to one or more embodiments of the invention. It is noted that the probe of the third aspect of the invention may be the same medical device (100) of the first aspect of the invention or a different medical device. It is therefore understood that any of the embodiments of the first aspect of the invention may comprise features according to any of the second aspect of the invention in any combination possible and vice versa.

The device (300) is essentially elongated, so that it is configured to receive a probe longitudinally (not shown). It should be noted that while Figs. 18A and 18B describe a device (300) with a certain length and a certain length-to-width ratio, the device (300) of the present invention is not limited to any particular length. Likewise, it can be seen that the device (300) of Figs. 18A and 18B comprises an irregular shape on the outside, however, the invention is not limited in this sense and in other embodiments it could have different shapes on the outside, as well as be completely smooth.

The device (300) comprises a proximal zone and a distal zone. The proximal zone comprises an opening (302) and an indentation (304). The opening (302) is configured for the insertion of the probe (not shown) to which the membrane (70) is to be arranged in such a way as to allow the insertion of the probe (not shown) into the device (300). Although Fig. 18A shows a circular opening, the opening (302) of the present invention is not limited in form. Thus, in other embodiments, the aperture may have different sizes and shapes that may vary depending on the shape of the probe (not shown) and the shape of the membrane (70) to be available as an expert in the field can appreciate. So, for example, the opening can be oval, quadrangular, or any other shape.

The indentation (304) is defined by the outer surface of the proximal area of the device (300) radially. The indentation (304) can form in a variety of ways, as a subject matter expert can appreciate. For example, the indentation may form as a groove on the outer surface of the device (300), or it may be formed by arranging one or more radial extensions of the surface in such a way that a radial indentation is formed on the proximal surface of the device (300). Very preferably, the indentation (304) will define a plane parallel to the opening (302). The indentation can be defined at different distances from the opening (302). Preferably, the indentation (304) will be defined at the end of the proximal area, next to the opening (302).

In addition, as can be seen in Fig. 18B, the indentation (304) is configured to receive the edge (74) of a membrane (70) covering the opening (302). The membrane (70) is essentially elongated, preferably essentially cylindrical, such that the edge (74) of the membrane (70) comprises part of the membrane (70). The membrane (70) to be received is essentially elongated, so that it can be adapted to the length and width of the probe (not shown) to be covered. As the edge (74) comprises part of the membrane (70), the membrane has an essentially flat configuration when placing its edge (74) in the indentation (304). Preferably, the edge (74) comprises part of the membrane (70) in a coiled manner such that the membrane (70) is wound internally, being comprised between the unfolded membrane (70) and the indentation (304) as shown in Fig. 18B. The indentation (304) is preferably defined with an essentially concave shape, so that it can receive an edge (74) with a preferably toroidal shape.

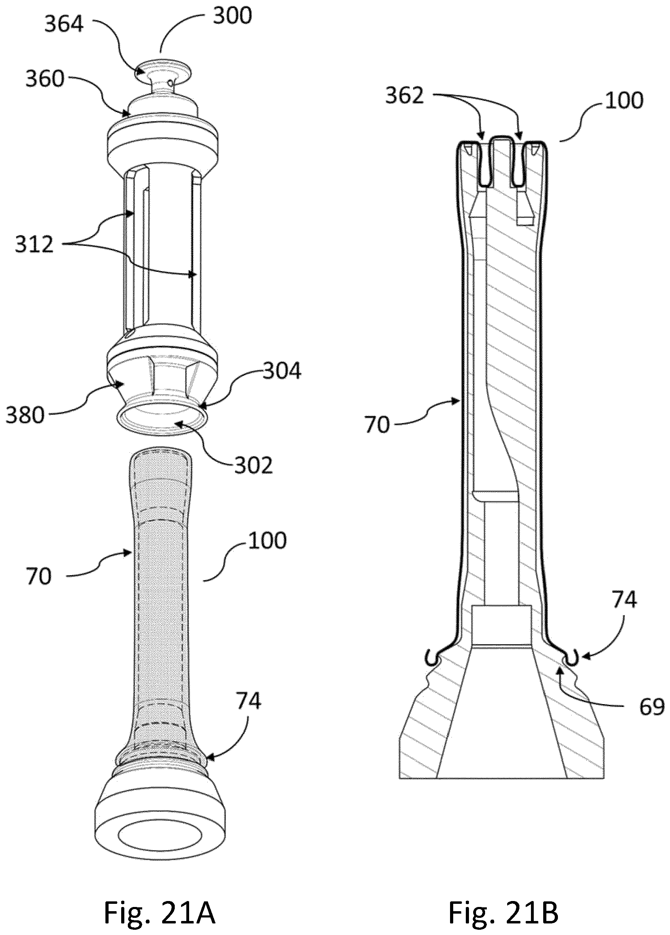

Advantageously, this design of the indentation (304) allows the device (300) to be configured so that once the membrane (70) is arranged in such a way that the edge (74) of the membrane (70) is received by the indentation (304), the membrane completely covers the opening (302), performing this entire process without touching the membrane at any time. ensuring its sterilization. Since the opening (302) is configured for the insertion of the probe (100), the insertion of the probe (100) involves forcing the arrangement of the membrane (70) onto the surface of the probe (100), as will be explained below with respect to Figs. 20A, 20B, 21A and 21B.

As can be seen, Figs. 18A and 18B show a device (300) that includes many other additional features, which may not be found or can be found differently in other embodiments. For example, in its distal area, device (300) comprises a circular handle-shaped surface (364) that allows the device to be held (10). However, this feature is optional, and other embodiments may not include it or may have a handle (364) with a different shape. Another characteristic is the presence of a series of lateral openings (12) that allow the correct arrangement of the membrane (70) on the probe (100) not shown to be observed. However, in other embodiments, the side openings (12) may not meet, have different shapes or the number of openings may be different. The device (300) also includes a series of extensions in the proximal and distal areas that allow better handling of the device (300) during use. However, in other embodiments, the device may not include these extensions or could have other shapes.

It can also be seen that the membrane (70) shown in Fig. 18B has an essentially circular shape. However, in other embodiments the device (300) may be configured to receive the edge of a membrane with a different shape, or with an irregular shape. In this way, device (300) according to the present invention can be configured to arrange membranes (70) of different shapes, suitable to be arranged on probes of different shapes. It can also be seen that the membrane (70) comprises a protrusion (72) in its center, which may be designed to optimally fit the probe (not shown). However, in later embodiments, the membrane (70) may not include this protrusion (72), or it may include it in another uncentered place, or it may have more than one protrusion (72) or have one or more protrusions (72) of different shape. Preferably, the membrane (70) comprises a lubricant on its outer surface, making it easier to introduce the probe (100) through a duct or body cavity.

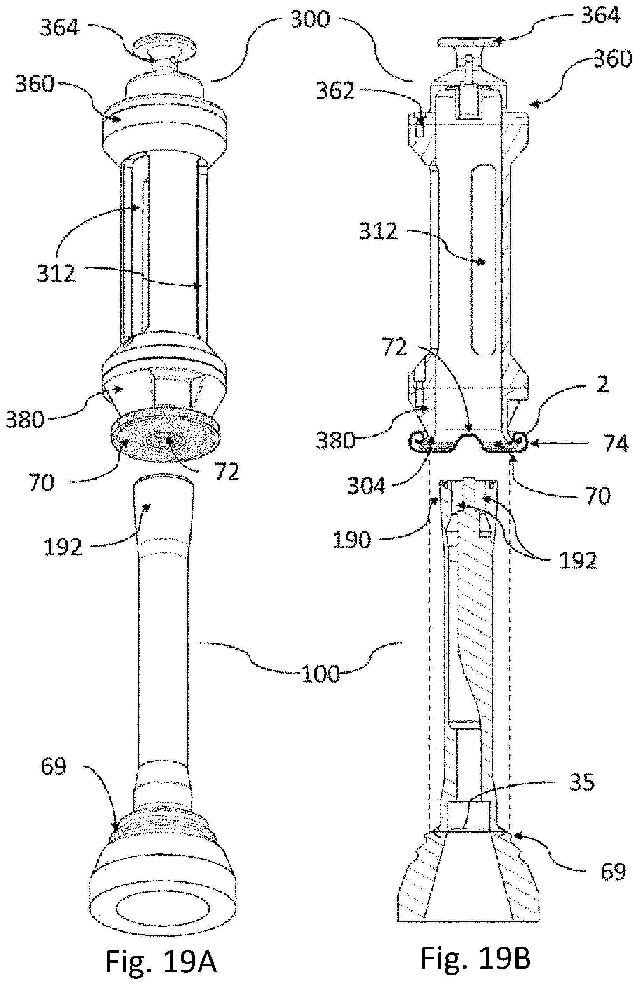

Figs. 19A and 19B show a perspective view and longitudinal section of the device (300) of Fig. 18A with a membrane (70) and a probe (100) for which it is configured to receive according to one or more embodiments of the invention.

The probe (100) is essentially tubular, and allows exploration, diagnosis, and/or provision of therapy through a duct or body cavity. It should be noted that the probe (100) in Figs. 19A and 19B has an internal surface, such that it is hollow inside, accessible from the proximal part opposite the distal end (190). However, in other embodiments, the probe (100) may be solid and not hollow or have a different design, for example, housing different elements inside it depending on the functions to be performed by the probe. Similarly, probe (100) has a widening in its proximal part. However, in other embodiments, probe (100) may not include this widening or may have others-shapes. Preferably, the probe (100) has a slit or indentation (69) in its proximal part, configured to receive the edge of the membrane (70) once it has been placed on the probe (100), as will be explained later with respect to Figs. 20A, 20B, 21A and 21B.

It should be noted that, as in Figs. 18A and 18B., the device (300) and the membrane (70) include other additional characteristics, which may not be found or may be found differently in other embodiments. Thus, the same considerations regarding alternative forms apply with respect to Figs. 19A and 19B.

In a preferred embodiment as shown in Figs. 19A and 19B, the probe (100) comprises a distal end (190) through which it is introduced into the device (300). The distal end (190) of the probe (100) can come in a variety of shapes and sizes, depending on the catheter application. The probe (100) has a distal end with a specific shape, as shown in Fig. 19B, but in other embodiments the shape of the distal end (190) may be different.

In this preferred embodiment, the device (300) also comprises a distal end (360) in its distal area, which in turn comprises an inner surface configured to receive the distal end (190) of the probe (100). Advantageously, this allows the membrane (70) to be inserted into the device (300) to be arranged snugly at the distal end (190) of the probe (100).

In an even more preferred embodiment, as shown in Figs. 19A and 19B, the inner surface of the distal end (360) of the device (300) comprises the complementary shape of the distal end (190) of the probe (100), configured to arrange the membrane (70) in contact with the distal end (190) of the probe (100) uniformly along the entire surface of the distal end (190) of the probe (100).

Figs. 20A and 20B show a perspective view and longitudinal section of the device in Fig. 18A, with the probe fully inserted and the membrane fully arranged on it, according to one or more embodiments of the invention. It should be noted that, as in Figs. 18A, 18B, 19A and 19B, the device (300), the membrane (70) and the probe (100) comprise additional characteristics, which may not be found or may be found differently in other embodiments. Thus, the same considerations regarding alternative forms apply with respect to Figs. 20A and 20B.

As can be seen in Figs. 20A and 20B, when the probe (100) is inserted through the opening (302), the membrane (70) slides along the probe (100), and because the inner surface of the distal end (360) of the device (300) comprises the complementary shape of the distal end (190) of the probe (100), The membrane is arranged evenly between the device (300) and the probe (100) along the entire surface of the distal end (190) of the probe (100).

In the case of device (300) in Figs. 19A, 19B, 20A and 20B, the membrane is arranged in a characteristic pattern, which in the longitudinal section of Fig. 20B is seen as an indentation (192). This is the shape of the surface of the distal end (190) of the probe (100) according to the example in the reference figures. However, it can be seen that, in other embodiments, the probe (100) may comprise a distal end (190) with a differently shaped surface, with or without an indentation (192), so that the device (300), in accordance with this preferential embodiment, will have another shape, corresponding to the complementary shape of the distal end (190) of the probe (100). Thus, the membrane should also be arranged in such a way that it is evenly distributed over the surface of the probe (100), especially on the surface of the distal end (190) of the probe (100).

Therefore, advantageously, this preferred embodiment allows the membrane (70) to be evenly distributed over the probe (100) when it is inserted, even when the distal end (190) of the probe (100) is irregularly shaped.

As can be seen in Figs. 20A and 20B, the membrane (70), when unfolded from its flat shape, acquires an elongated shape, preferably in an essentially cylindrical shape. The edge (74) of the membrane (70) which included part of the membrane (70) barely comprises part of the membrane (70), since it has been unfolded as the probe (100) has been introduced into the device (300). In this way, the indentation (304) of the device (300) contains only part of the membrane, since almost the entire edge has been unfolded. In a preferred embodiment where the edge (74) comprises part of the membrane (70) in a coiled form, the edge (74) unfolds by unwinding.

According to another preferred embodiment of the invention, the distal end (360) of the device (300) is configured to be decoupled from the rest of the device (300) and replaced by another distal (not shown) end of the device (300) according to the distal end (190) of the probe (100). Advantageously, this allows the same device (300) to be compatible with different probes (100) and to arrange a membrane (70) on them regardless of their shape or size.

In this preferential implementation, the device (300) is designed to be able to arrange membranes (70) on different probes (100), regardless of their length and the shape of their distal end (190). When device (300) has a distal end (360) configured for a probe other than the probe (100) to which we want to attach the membrane (70), we can decouple the distal end (360) from the device (300) and replace it with another distal end (not shown) that fits the probe (100).

It should be noted that the distal end (360) can be adapted to have different lengths, widths and shapes depending on the probe (100) as the person skilled in the field can appreciate. Thus, if, for example, probe (100) is longer than the probe for which the distal end (360) is configured, it can be replaced by a new distal end that is longer than the current distal end (360). Similarly, if the probe (100) is of a different width or shape than the probe for which the distal end (360) is configured, it may be replaced by a new distal end with a different width or shape than the current distal end (360).

It is also noted that it is possible to decouple and attach the distal end (360) of the device (300) in a variety of ways. Although the device (300) in Figs. 19B and 20B is shown to be attached to the rest of the device (300) by means of a screw (62), the expert in the field can observe that there are multiple alternatives by which in other embodiments the distal end (360) of the device (300) is used. For example, it is possible that the distal end (360) has a threaded surface such that it is mated by screwing the distal end (360) into the distal area of the device (300). Alternatively, the distal end (360) snaps into the distal area of the device (300). All these alternatives are also part of the present invention.

According to another preferred embodiment of the invention, the opening (302) is set to conform to the respective surface of the probe (100) when the probe (100) is fully inserted into the device (300). As shown in Figs. 20A and 20B, device (300) has an opening (302), where the aperture has a certain shape that conforms to the respective surface of the probe (100) when probe (100) is fully inserted into the device (300).

Advantageously, this means that when the membrane (70) is completely arranged on the surface of the probe (100), which occurs when the probe (100) is fully inserted into the device (300), the membrane (70) is closely attached to the surface of the probe (100), which favors its attachment in the designated place.

More preferably, when probe (100) has a circular section such as to define a diameter (35) as shown in Figs. 18A, 19B and 20B, the opening (302) is set to conform to the diameter (35) of the probe (100) surface at the respective position when probe (100) is fully inserted into the device (300). In these figures it can be seen that the diameter of the opening (302) of the device (300) is equal to the diameter (35) of the probe (100) at the position of the probe (100) that is at the height of the opening (302) when the probe (100) is fully inserted into the device (300).

It should be noted that, although device (300) and probe (100) in Figs. 19A, 19B, 20A and 20B are configured so that the position of the probe (100) at the opening (302) when the probe (100) is fully inserted into the device (300) is wider than the rest of the probe (100), In other embodiments, it is possible that the width of the probe (100) at this height is equal to the rest of the probe.

In another preferred embodiment of the invention, the indentation (304) is configured to allow the release of the membrane (70) once the probe (100) has been inserted to the distal end (360) of the device (300). Once the probe (100) has been inserted to the distal end (360) of the device (300), the edge (74) of the membrane (70) barely comprises part of the membrane, since it has been unfolded as the probe (100) has been inserted into the device (300). According to the figures in question, the indentation (304) of the device (300) comprises an inclined surface which, once the edge has been unfolded, hardly exerts any resistance to the release of the edge (74) of the indentation (304). Thus, by removing the probe (100) from the device (300), the edge (74) of the membrane (70) can be released from the indentation (304) of the device (300). It should be noted that in other embodiments the slit may be configured to allow the release of the membrane (70) once the probe (100) has been introduced to the distal end (360) of the device (300), as the expert in the field can appreciate. For example, in other embodiments, device (300) may have a flexible or retractable indentation (304), configured so that when probe (100) is inserted to the distal end (360) of the device (300), it no longer receives the edge (74) of the membrane.

Advantageously, this allows the same gesture of inserting the probe (100) into the device (300), which, as explained, arranges the membrane (70) on the surface of the probe (100) homogeneously, also frees the membrane (70) from the indentation (304), making the system simple and efficient.

Figs. 21A and 21B show a perspective view and longitudinal section of the device in Fig. 18A and a probe once it has been removed from the device, according to one or more embodiments of the invention. It should be noted that, as in Figs. 18A, 18B, 19A, 19B, 20A and 20B, the device (300), the membrane (70) and the probe (100) comprise additional features, which may not be found or may be found differently in other embodiments. Thus, the same considerations regarding alternative forms apply with respect to Figs. 21A and 21B.

In another preferred embodiment of the invention, the device (300) is configured to arrange the membrane (70) in a probe (100) which in turn comprises in its proximal part a slit or indentation (69) configured to receive the edge (74) of the membrane (70) once it has already been placed on the probe (100).

The indentation (69), like the indentation (304), can be formed in a variety of ways, as an expert in the field can appreciate. For example, the indentation may form as a groove on the outer surface of the device (300), or it may be formed by arranging one or more radial extensions of the surface in such a way that a radial indentation is formed on the proximal surface of the device (300). Very preferably, the indentation (69) will define a plane parallel to the longitudinal axis of the probe (100). The slit can be defined at different distances from the position of the probe (100) at the opening (302) when the probe (100) is fully inserted into the device (300). Preferably, the slit (3 4) will be defined close to the position of the probe (100) that is at the level of the opening (302) when the probe (100) is fully inserted into the device (300).

As shown in Figs. 21A and 21B, this advantageously allows the edge (74) of the membrane (70) to be fixed in the indentation (69) of the probe (100) when the probe (100) is removed from the device.

In an even more preferred embodiment of the invention, as shown in Figures 19A, 19B, 20A, 20B, 21A and 21B, the indentation (304) is configured to allow the release of the membrane (70) once the probe (100) has been introduced to the distal end (360) of the device (300) and the probe (100) in turn comprises in its proximal part a slit or indentation (69) configured to receive the edge of the membrane (70) once it has been placed on the probe (100). Advantageously, this allows the membrane (70) to be placed homogeneously, consistently and quickly with a gesture of inserting and removing the probe (100) into the device (300) to which a membrane (70) has been placed over the probe (100) over its opening (302).

In a preferred embodiment of the invention, as shown in Figs. 18A, 18B, 19A, 19B, 20A, 20B, 21A, and 21B, the opening (302) is essentially circular. As shown in the figures, the opening (302) is circular, but in other embodiments according to the present invention, the opening (302) may have other shapes, as the expert in the art can appreciate. For example, the opening (302) may have an essentially circular shape and be slightly oval in some of its parts, or if the circular opening (302) lacks a segment of it. Also if, for example, in order to orient the insertion of the probe in a correct position and avoid rotation during its insertion, the essentially circular opening (302) may have a notch on its surface configured to channel another notch included in the probe (100) in such a way that the probe (100) can only be inserted through the opening (302) in a specific position. Therefore, the expert in the art can envisage various ways of making the opening (302) essentially circular, all of which are covered by the present invention.

In addition, in accordance with this preferred embodiment, the indentation (304) defines a circumference on the surface of the proximal zone with a diameter between 5 and 50 mm, more preferably between 10 and 40 mm.

Advantageously, this preferred embodiment allows the membrane (70) to be essentially elongated and essentially cylindrical, allowing the use of various common medical-grade membranes, such as prophylactics. The fact that the opening (302) is essentially circular, as described above, allows commercial membranes to be arranged in a regular and fluid manner when the probe (100) is inserted through the opening (302), as these usually have an essentially circular cross-section. The diameter being between 5 and 50 mm allows the device (300) to be configured to receive common medical-grade membranes in its indentation (304) covering the opening (302) according to various commonly used sizes, such as commercial and prophylactic sizes.

In another preferred embodiment of the invention, as shown in Figs. 18A, 18B, 19A, 19B, 20A, 20B, 21A and 21B, the membrane (70) is a prophylactic. Advantageously, this allows the device (300) to be configured to deliver membranes (70) in a probe (100) quickly and efficiently, at low cost and easy to obtain. Preferably, the membrane (70) additionally includes a lubricant on its outer surface, facilitating the insertion of the probe (100) through a duct or cavity of the body.

In another preferred embodiment of the invention, as shown in Figs. 18A, 18B, 19A, 19B, 20A and 21A, the device comprises at least one lateral aperture (12) configured to allow visualization of the membrane (70) arrangement over the probe (100). It should be noted that, although the device (300) in the figures cited comprises three (12) lateral openings, in other embodiments in accordance with the present invention, the device (300) may comprise one, two or more of three (12) lateral openings, in order to allow visualization of the arrangement of the membrane (70) over the probe (100). Similarly, it can be observed that in the figures the lateral openings (12) have an elongated shape, parallel to the longitudinal axis of the device (300) and are essentially rectangular with rounded corners. In addition, the three side openings (12) are arranged equi-spaced on the surface of the device (300) parallel to each other. However, the subject matter expert can appreciate that there are multiple ways to design and arrange at least one lateral opening (312) on the surface of the device (300). In this sense, at least one lateral opening (312) may have any other shape, length and arrangement, and when more than one opening is included in the device (300), it is possible that they are irregularly or regularly arranged on it and it is possible that they have the same or different shape. Thus, all these possible alternatives of designing at least one lateral opening (312) configured to allow visualization of the arrangement of the membrane (70) over the probe (100) are included in this preferred embodiment of the invention.

Advantageously, being able to visualize the arrangement of the membrane (70) on the probe (100) allows to verify that it is being inserted correctly into the device (100), so that no folds or irregularities are formed in the membrane (70) that prevent an inhomogeneous arrangement of the membrane (70) on the probe (30 ), and thus to be able to correct the insertion of the probe (100) into the device (300) as it is introduced to ensure a homogeneous arrangement of membrane (70).

In another preferred embodiment, as shown in Figs. 19A, 19B, 20A, 20B, 21A, and 21B, device (300) is configured to arrange the membrane (70) in a probe (100), more preferably in a uterine probe. As can be seen in the figures, the probe (100) is a uterine probe, where its distal end (190) is configured to receive the cervix from a patient's uterus, through a design that includes an indentation (192) that allows the uterine cervix to be contacted once the probe is introduced into the vagina. It should be noted that the probe (100) in Figs. 19A, 19B, 20A, 20B, 21A and 21B is merely representative, and that the uterine probe (100) can take different shapes, so that it is able to interact by different means, for different applications and/or with different orientations. Advantageously, this preferred method allows the same uterine probe (100) to be used in several examinations, either in the same or in different patients, simply by replacing the membrane (70) that is placed on the probe (100) in each of the examinations performed.

In an even more preferred embodiment of this embodiment, device (300) is configured to arrange the membrane (70) in a uterine torsional wave probe (100) in such a way that it comprises at its distal end (190) a torsional wave emitter and receiver. Preferably, the uterine torsional wave probe (100) is configured to characterize the structure of the uterine cervix. Advantageously, this allows the use of the same uterine torsional wave probe (100) in different examinations in the same or different patients, simply by replacing the membrane (70) that is placed on the probe (100) in each of the examinations performed. Even more preferably, the uterine torsional wave probe (100) is a torsional ultrasonic wave uterine probe (100).

In another preferred embodiment of the invention, as shown in Figs. 18A, 18B, 19A, 19B, 20A, 20B, and 21A, device (300) comprises at its distal end (360) a handle (364). Advantageously, this allows that, once the probe (100) has been completely inserted into the device (300), the probe (100) can be separated from the device (300), separating the device (300) in the distal direction.

In another preferred embodiment of the invention, the device (300) also comprises in its proximal area at least one magnet configured to attract at least one ferromagnetic element or magnet in the proximal part of the probe (100). Advantageously, this allows that once the probe (100) is inserted into the device (300), the device (300) and the probe (100) are joined through the magnetic interaction between at least one magnet of the device (300) and the at least one ferromagnetic element or magnet of the probe (100) facilitating the manipulation of the union of the two more easily. Preferably, the magnetic interaction is strong enough to allow the opposite element to be disengaged without applying a force by holding either the device (300) or the probe (100). In practice, this means that the device-probe assembly (100) can be operated with one hand, freeing the other hand for other uses, e.g. to ensure that the membrane (70) is correctly arranged on the probe (100).

In addition, this also makes it possible to define the correct way to orient the probe (100) when it is inserted into the device (300) by means of a specific arrangement of magnets (10). For example, device (300) may comprise at least one magnet arranged in a particular position that will attract at least one ferromagnetic element or magnet located at a particular location on the probe (100) to that particular position in which it is located, effectively aligning both positions.

At least one magnet can be made of various materials, as the expert in the field can appreciate. For example, the magnet may comprise one or more neodymium, or magnetite. In addition, at least one magnet can comprise various shapes: circular, rectangular, cubic, without being limited to any particular shape.

All of the above are fully within the scope of the present disclosure, and are considered to form the basis for alternative embodiments in which one or more combinations of the above described features are applied, without limitation to the specific combination disclosed above. In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art.

## Claims

1. Medical device (100) configured to contact a specimen (200) through its distal end (190), the medical device (100) comprising :
a. A torsional ultrasonic waves transducer (10) located at the distal end (190) of the medical device (100) and configured to induce a torsional wave that goes through said specimen (200),
b. a force sensor (20) configured to determine the force with which the medical device (100) contacts the specimen (200), and
c. a processing unit (30);
wherein the torsional ultrasonic waves transducer (10) is configured to contact the specimen (200) with a predetermined range of force,
wherein the processing unit (30) is configured to determine whether the force with which the medical device (100) contacts the specimen (200) is within the predetermined range of force; and
wherein the specimen (200) is the cervix of a uterus and the torsional ultrasonic waves transducer (10) is configured to contact the cervix (200) with a range between 0 and 200 g/cm².

2. The medical device (100) according to claim 1, further comprising feedback means (40), wherein the feedback means (40) are configured to provide feedback to the user of the medical device (100) about the force with which the device contacts the specimen (200).

3. The medical device (100) according to claim 2, wherein the feedback means (40) are configured to further provide feedback to the user of the medical device (100) about whether the force is within the predetermined range of force or not.

4. The medical device (100) according to any one of claims 2 or 3, further comprising an imaging means (41), wherein the feedback means (40) comprise a visual feedback device configured to display the images captured by the imaging means (41).

5. The medical device (100) according to any one of the previous claims, wherein the force sensor (20) is comprised within the distal section (180) of the medical device (100).

6. The medical device (100) according to any one of the previous claims, wherein the force sensor (20) is located perpendicularly to the surface of the medical device (100) configured to contact the specimen (200).

7. The medical device (100) according to any one of the previous claims, wherein the force sensor (20) further comprises a protection system configured to prevent the force sensor (20) from getting damaged when a force exceeding the working range is applied to the distal end (190) of the medical device (100), preferably wherein the protection system comprises a spring (21).

8. The medical device (100) according to any of the previous claims, further comprising a handle (50) on its proximal end (110) configured to be held by a hand and with an angle (α) comprised between 85° and 120° with respect to the axis (130) formed by the distal end (190) and proximal end (110).

9. The medical device (100) according to any of the previous claims, wherein the processing unit (30) is configured to discard the readings of torsional ultrasonic waves transducer (10) if the force applied is not within the predetermined range of force.

10. The medical device (100) according to any of the previous claims, wherein the processing unit (30) is configured to prevent the torsional ultrasonic waves transducer (10) from inducing torsional waves if the force applied is not within the predetermined range of force.

11. The medical device (100) according to any of the previous claims, wherein the torsional ultrasonic waves transducer (10) comprises an emitter device for emitting torsional ultrasonic waves, wherein the emitter device comprises an electrical signal generator (13) connected to an electromechanical actuator (12) which is in turn attached to a contact element (11) that comes into contact with a specimen (200), such that when the actuator receives electrical signals, it induces rotational movement of the contact element (11), and when said contact element (11) comes into contact with the specimen (200), it induces a torsional wave that goes through said specimen (200), wherein the torsional ultrasonic waves transducer (10) further comprises means for receiving the distorted signal after it goes through the specimen (200).

12. The medical device (100) according to claim 11, wherein the means for receiving the distorted signal comprise two or more piezoelectric elements (15) located equidistant from one another and placed between two rings (14a and 14b) made from a non-conducting material and in that the axis of rotation of the rings coincides with the axis of rotation of the electromechanical actuator (12).

13. The medical device (100) according to any of the previous claims, wherein the medical device (100) further comprises:
d. a conduit (62) to connect the outside to the inner space (120) of the medical device (100), the conduit (62) connected to the outside through an opening (63),
e. a sealing element (64) at the opposite end of the conduit (62), configured to control the passage of air between the inner space (120) and the conduit (62), and
f. a vacuum pump (65) connected to the sealing element (64) and configured to draw air from the opening (63) through the conduit (62);
wherein the medical device (100) is configured to receive a membrane (70) on its outside covering the opening (63), so that the vacuum pump (65) is configured to extract the air between the sealing element (64) and the membrane (70).

14. The medical device (100) according to claim 13, wherein the vacuum pump (65) is connected to a pressure sensor (67) configured to measure the pressure in the conduit (62), and where the vacuum pump (65) is configured to maintain a minimum pressure in the space between the membrane (70) and the sealing element (64) as measured by the pressure sensor (67).

15. The medical device (100) according to any of the previous claims wherein the medical device (100) is a probe.

## Patentansprüche

1. Medizinische Vorrichtung (100), welche dazu ausgebildet ist, über deren distalen Ende (190) mit einer Probe (200) in Kontakt zu kommen, wobei die medizinische Vorrichtung (100) Folgendes umfasst:
a. einen Torsionsultraschallwandler (10), welcher sich am distalen Ende (190) der medizinischen Vorrichtung (100) befindet und dazu ausgebildet ist, eine Torsionswelle zu induzieren, welche durch die genannte Probe (200) geht,
b. einen Kraftsensor (20), welcher dazu ausgebildet ist, die Kraft, mit welcher die medizinische Vorrichtung (100) mit der Probe (200) in Kontakt kommt, zu bestimmen, und
c. eine Verarbeitungseinheit (30);
wobei der Torsionsultraschallwandler (10) dazu ausgebildet ist, mit einem vorbestimmten Kraftbereich mit der Probe (200) in Kontakt zu kommen,
wobei die Verarbeitungseinheit (30) dazu ausgebildet ist, zu bestimmen, ob die Kraft, mit welcher die medizinische Vorrichtung (100) mit der Probe (200) in Kontakt kommt, innerhalb des vorbestimmten Kraftbereichs liegt; und
wobei die Probe (200) der Hals einer Gebärmutter ist und der Torsionsultraschallwandler (10) dazu ausgebildet ist, mit einem Bereich zwischen 0 und 200 g/cm² mit dem Hals (200) in Kontakt zu kommen.

2. Medizinische Vorrichtung (100) nach Anspruch 1, zusätzlich umfassend Rückmeldungsmittel (40), wobei die Rückmeldungsmittel (40) dazu ausgebildet sind, dem Benutzer der medizinischen Vorrichtung (100) eine Rückmeldung über die Kraft, mit welcher die Vorrichtung mit der Probe (200) in Kontakt kommt, bereitzustellen.

3. Medizinische Vorrichtung (100) nach Anspruch 2, wobei die Rückmeldungsmittel (40) dazu ausgebildet sind, zusätzlich dem Benutzer der medizinischen Vorrichtung (100) eine Rückmeldung darüber bereitzustellen, ob die Kraft innerhalb des vorbestimmten Kraftbereichs liegt oder nicht.

4. Medizinische Vorrichtung (100) nach einem der Ansprüche 2 oder 3, zusätzlich umfassend ein Bildgebungsmittel (41), wobei die Rückmeldungsmittel (40) eine visuelle Rückmeldungsvorrichtung umfassen, welche dazu ausgebildet ist, die vom Bildgebungsmittel (41) erfassten Bilder anzuzeigen.

5. Medizinische Vorrichtung (100) nach einem der vorhergehenden Ansprüche, wobei der Kraftsensor (20) innerhalb des distalen Abschnitts (180) der medizinischen Vorrichtung (100) umfasst ist.

6. Medizinische Vorrichtung (100) nach einem der vorhergehenden Ansprüche, wobei sich der Kraftsensor (20) senkrecht zur Oberfläche der medizinischen Vorrichtung (100) befindet, welche dazu ausgebildet ist, mit der Probe (200) in Kontakt zu kommen.

7. Medizinische Vorrichtung (100) nach einem der vorhergehenden Ansprüche, wobei der Kraftsensor (20) zusätzlich ein Schutzsystem umfasst, welches dazu ausgebildet ist, zu verhindern, dass der Kraftsensor (20) geschädigt wird, wenn eine Kraft, welche den Arbeitsbereich überschreitet, auf das distale Ende (190) der medizinischen Vorrichtung (100) angewendet wird, wobei vorzugsweise das Schutzsystem eine Feder (21) umfasst.

8. Medizinische Vorrichtung (100) nach einem der vorhergehenden Ansprüche, zusätzlich umfassend einen Griff (50) an deren proximalen Ende (110), welcher dazu ausgebildet ist, mittels einer Hand und mit einem Winkel (α), welcher zwischen 85° und 120° in Bezug auf die Achse (130) gebildet durch das distale Ende (190) und das proximale Ende (110) umfasst ist, gehalten zu werden.

9. Medizinische Vorrichtung (100) nach einem der vorhergehenden Ansprüche, wobei die Verarbeitungseinheit (30) dazu ausgebildet ist, die Ablesungen des Torsionsultraschallwandlers (10) auszuschließen, wenn die angewendete Kraft nicht innerhalb des vorbestimmten Kraftbereichs liegt.

10. Medizinische Vorrichtung (100) nach einem der vorhergehenden Ansprüche, wobei die Verarbeitungseinheit (30) dazu ausgebildet ist, zu verhindern, dass der Torsionsultraschallwandler (10) Torsionswellen induziert, wenn die angewendete Kraft nicht innerhalb des vorbestimmten Kraftbereichs liegt.

11. Medizinische Vorrichtung (100) nach einem der vorhergehenden Ansprüche, wobei der Torsionsultraschallwandler (10) eine Sendevorrichtung zum Aussenden von Torsionsultraschallwellen umfasst, wobei die Sendevorrichtung einen elektrischen Signalgenerator (13) umfasst, welcher mit einem elektromechanischen Aktuator (12) verbunden ist, welcher wiederum an einem Kontaktelement (11) befestigt ist, welches mit einer Probe (200) in Kontakt kommt, sodass, wenn der Aktuator elektrische Signale empfängt, er eine Rotationsbewegung des Kontaktelements (11) induziert, und wenn das genannte Kontaktelement (11) mit der Probe (200) in Kontakt kommt, er eine Torsionswelle induziert, welche durch die genannte Probe (200) geht, wobei der Torsionsultraschallwandler (10) zusätzlich Mittel zum Empfangen des verzerrten Signals, nachdem es durch die Probe (200) geht, umfasst.

12. Medizinische Vorrichtung (100) nach Anspruch 11, wobei die Mittel zum Empfangen des verzerrten Signals zwei oder mehr piezoelektrische Elemente (15) umfassen, welche sich äquidistant zueinander befinden und zwischen zwei Ringen (14a und 14b), hergestellt aus einem nichtleitenden Material, platziert sind, und wobei die Rotationsachse der Ringe mit der Rotationsachse des elektromechanischen Aktuators (12) übereinstimmt.

13. Medizinische Vorrichtung (100) nach einem der vorhergehenden Ansprüche, wobei die medizinische Vorrichtung (100) zusätzlich Folgendes umfasst:
d. eine Leitung (62) zum Verbinden des Äußeren mit dem Innenraum (120) der medizinischen Vorrichtung (100), wobei die Leitung (62) mit dem Äußeren über eine Öffnung (63) verbunden ist,
e. ein Dichtelement (64) am gegenüberliegenden Ende der Leitung (62), welches dazu ausgebildet ist, den Durchgang von Luft zwischen dem Innenraum (120) und der Leitung (62) zu kontrollieren, und
f. eine Vakuumpumpe (65), welche mit dem Dichtelement (64) verbunden ist und dazu ausgebildet ist, Luft aus der Öffnung (63) durch die Leitung (62) zu ziehen;
wobei die medizinische Vorrichtung (100) dazu ausgebildet ist, eine Membran (70) auf deren Außenseite aufzunehmen, welche die Öffnung (63) deckt, sodass die Vakuumpumpe (65) dazu ausgebildet ist, die Luft zwischen dem Dichtelement (64) und der Membran (70) zu entnehmen.

14. Medizinische Vorrichtung (100) nach Anspruch 13, wobei die Vakuumpumpe (65) mit einem Drucksensor (67) verbunden ist, welcher dazu ausgebildet ist, den Druck in der Leitung (62) zu messen, und wobei die Vakuumpumpe (65) dazu ausgebildet ist, einen Mindestdruck im Raum zwischen der Membran (70) und dem Dichtelement (64), wie mittels des Drucksensors (67) gemessen wird, aufrechtzuerhalten.

15. Medizinische Vorrichtung (100) nach einem der vorhergehenden Ansprüche, wobei die medizinische Vorrichtung (100) eine Sonde ist.

## Revendications

1. Dispositif médical (100) configuré pour entrer en contact avec un échantillon (200) par son extrémité distale (190), le dispositif médical (100) comprenant :
a. un transducteur d'ondes ultrasonores de torsion (10) situé dans l'extrémité distale (190) du dispositif médical (100) et configuré pour induire une onde de torsion qui traverse ledit échantillon (200),
b. un capteur de force (20) configuré pour déterminer la force avec laquelle le dispositif médical (100) entre en contact avec l'échantillon (200), et
c. une unité de traitement (30) ;
dans lequel le transducteur d'ondes ultrasonores de torsion (10) est configuré pour entrer en contact avec l'échantillon (200) avec une plage de force prédéterminée,
dans lequel l'unité de traitement (30) est configurée pour déterminer si la force avec laquelle le dispositif médical (100) entre en contact avec l'échantillon (200) se trouve dans la plage de force prédéterminée ; et
dans lequel l'échantillon (200) est le col utérin et le transducteur d'ondes ultrasonores de torsion (10) est configuré pour entrer en contact avec le col utérin (200) avec une plage comprise entre 0 et 200 g/cm².

2. Dispositif médical (100) selon la revendication 1, comprenant en outre des moyens de rétroaction (40), dans lequel les moyens de rétroaction (40) sont configurés pour fournir une rétroaction à l'utilisateur du dispositif médical (100) concernant la force avec laquelle le dispositif entre en contact avec l'échantillon (200).

3. Dispositif médical (100) selon la revendication 2, dans lequel les moyens de rétroaction (40) sont configurés pour fournir en outre une rétroaction à l'utilisateur du dispositif médical (100) indiquant si la force se trouve dans la plage de force prédéterminée ou non.

4. Dispositif médical (100) selon l'une quelconque des revendications 2 ou 3, comprenant en outre des moyens d'imagerie (41), dans lequel les moyens de rétroaction (40) comprennent un dispositif de rétroaction visuel configuré pour afficher les images captées par les moyens d'imagerie (41).

5. Dispositif médical (100) selon l'une quelconque des revendications précédentes, dans lequel le capteur de force (20) est compris dans la section distale (180) du dispositif médical (100).

6. Dispositif médical (100) selon l'une quelconque des revendications précédentes, dans lequel le capteur de force (20) est situé perpendiculairement à la surface du dispositif médical (100) configuré pour entrer en contact avec l'échantillon (200).

7. Dispositif médical (100) selon l'une quelconque des revendications précédentes, dans lequel le capteur de force (20) comprend en outre un système de protection configuré pour empêcher le capteur de force (20) d'être endommagé lorsqu'une force dépassant la plage de travail est appliquée sur l'extrémité distale (190) du dispositif médical (100), de préférence dans lequel le système de protection comprend un ressort (21).

8. Dispositif médical (100) selon l'une quelconque des revendications précédentes, comprenant en outre une poignée (50) sur son extrémité proximale (110) configurée pour être tenue par la main et avec un angle (α) compris entre 85° et 120° par rapport à l'axe (130) formé par l'extrémité distale (190) et l'extrémité proximale (110).

9. Dispositif médical (100) selon l'une quelconque des revendications précédentes, dans lequel l'unité de traitement (30) est configurée pour rejeter les lectures du transducteur d'ondes ultrasonores de torsion (10) si la force appliquée ne se trouve pas dans la plage de force prédéterminée.

10. Dispositif médical (100) selon l'une quelconque des revendications précédentes, dans lequel l'unité de traitement (30) est configurée pour empêcher le transducteur d'ondes ultrasonores de torsion (10) d'induire des ondes de torsion si la force appliquée ne se trouve pas dans la plage de force prédéterminée.

11. Dispositif médical (100) selon l'une quelconque des revendications précédentes, dans lequel le transducteur d'ondes ultrasonores de torsion (10) comprend un dispositif émetteur pour émettre des ondes ultrasonores de torsion, dans lequel le dispositif émetteur comprend un générateur de signaux électriques (13) relié à un actionneur électromécanique (12) qui est à son tour relié à un élément de contact (11) qui entre en contact avec un échantillon (200) de telle sorte que, lorsque l'actionneur reçoit des signaux électriques, il induit un mouvement de rotation de l'élément de contact (11), et lorsque ledit élément de contact (11) entre en contact avec l'échantillon (200), il induit une onde de torsion qui traverse ledit échantillon (200), dans lequel le transducteur d'ondes ultrasonores de torsion (10) comprend en outre des moyens pour recevoir le signal distordu après avoir traversé l'échantillon (200).

12. Dispositif médical (100) selon la revendication 11, dans lequel les moyens pour recevoir le signal distordu comprennent deux ou plusieurs éléments piézoélectriques (15) situés équidistants les uns des autres et placés entre deux anneaux (14a et 14b) faits en un matériau non conducteur et en ce que l'axe de rotation des anneaux coïncide avec l'axe de rotation de l'actionneur électromécanique (12).

13. Dispositif médical (100) selon l'une quelconque des revendications précédentes, dans lequel le dispositif médical (100) comprend en outre :
d. un conduit (62) pour relier l'extérieur à l'espace intérieur (120) du dispositif médical (100), le conduit (62) étant relié à l'extérieur à travers une ouverture (63),
e. un élément de scellement (64) dans l'extrémité opposée du conduit (62), configuré pour contrôler le passage d'air entre l'espace intérieur (120) et le conduit (62), et
f. une pompe à vide (65) reliée à l'élément de scellement (64) et configurée pour aspirer de l'air à partir de l'ouverture (63) à travers le conduit (62) ;
dans lequel le dispositif médical (100) est configuré pour recevoir une membrane (70) sur la partie extérieure de celui-ci recouvrant l'ouverture (63), de sorte que la pompe à vide (65) est configurée pour extraire l'air entre l'élément de scellement (64) et la membrane (70).

14. Dispositif médical (100) selon la revendication 13, dans lequel la pompe à vide (65) est reliée à un capteur de pression (67) configuré pour mesurer la pression dans le conduit (62), et où la pompe à vide (65) est configurée pour maintenir une pression minimale dans l'espace entre la membrane (70) et l'élément de scellement (64) telle que mesurée par le capteur de pression (67).

15. Dispositif médical (100) selon l'une quelconque des revendications précédentes, dans lequel le dispositif médical (100) est une sonde.
